# EUROPEAN PATENT APPLICATION

(11) **EP 3 150 206 A1**
(43) Date of publication of application: **05.04.2017**
(21) Application number: 15799603.4
(22) Date of filing: 27.05.2015
(51) Int. Cl.: A61K 31/497, A61P 35/00, A61P 35/02

(54) **MEDICINAL COMPOSITION COMPRISING PYRAZINE CARBOXAMIDE COMPOUND AS ACTIVE INGREDIENT**

(30) Priority: 28.05.2014 JP 2014109952
(71) Applicant: Astellas Pharma Inc., Chuo-ku Tokyo 103-8411 (JP)
(72) Inventor: KONAGAI, Satoshi, Tokyo 103-8411 (JP); TANAKA, Hiroaki, Tokyo 103-8411 (JP); YAMAMOTO, Hiroko, Tokyo 103-8411 (JP); SAKAGAMI, Hideki, Tokyo 103-8411 (JP)
(74) Representative: Bates, Philip Ian
(86) International application number: PCT/JP2015/065154
(87) International publication number: WO 2015/182628

(57) **Abstract**

[Problem]

Provided is a pharmaceutical composition for treating cancer in which one or more kinases of BTK, JAK3, and ITK is involved.

[Means for Solution]

The present inventors studied compounds having a BTK inhibiting effect, a JAK3 inhibiting effect and an ITK inhibiting effect, and confirmed that a specific pyrazine carboxamide compound has the BTK inhibiting effect, the JAK3 inhibiting effect, and the ITK inhibiting effect, and that a pharmaceutical composition comprising the compound as an active ingredient has a therapeutic effect on cancer in which one or more kinases of BTK, JAK3 and ITK is involved, in another aspect, cancer in which BTK is overexpressed or activated, in another aspect, cancer in which a B cell receptor signal is activated, in still another aspect, cancer in which JAK3 is activation-mutated or activated, and in still another aspect, cancer in which ITK is activated, thereby completing the present invention.

## Description

### Technical Field

The present invention relates to a pharmaceutical composition for treating cancer comprising a pyrazine carboxamide compound or a pharmaceutically acceptable salt thereof as an active ingredient.

### Background Art

BTK (Bruton's tyrosine kinase) is a non-receptor type tyrosine kinase which belongs to the Tec family of kinases, and is a protein which has a pleckstrin homology domain, a Tec homology domain, a Src homology 2 domain, a Src homology 3 domain, and a kinase domain. BTK is expressed in most of hematopoietic cells excluding T cells and plasma cells, and is an essential molecule for production of B cells (Exp. Hematol. Oncol. 2014; 3(1):4, and J. Hematol. Oncol. 2013; 6:59). In B cells, BTK transfers a B cell receptor signal to downstream phospholipase C gamma and causes calcium ion mobilization and activation of a mitogen-activated protein kinase pathway or a nuclear factor kappa B (Proc. Natl. Acad. Sci. USA. 2010; 107(29):13075-13080).

Hitherto, it has been suggested that BTK is involved in the progression of B-cell malignant tumors including chronic lymphocytic leukemia, mantle cell lymphoma, follicular lymphoma, diffuse large B-cell lymphoma, and acute lymphocytic leukemia, and acute myeloid leukemia (Exp. Hematol. Oncol. 2014; 3(1):4, and Blood. 2014; 123(8):1229-1238). In addition, it has been reported that in a primarily cultured cell derived from a patient with acute myeloid leukemia, suppression of the expression of BTK by miRNA inhibits proliferation of cells, and that a BTK inhibitor inhibits proliferation of the diffuse large B-cell lymphoma cells in which a B cell receptor signal is activated (Blood. 2014; 123(8):1207-1213, and Nature. 2010; 463:88-92). Clinical development of BTK inhibitors have been conducted for targeting cancers including follicular lymphoma, chronic lymphocytic leukemia, small lymphocytic lymphoma, multiple myeloma, mantle cell lymphoma, diffuse large B-cell lymphoma, and B-cell prolymphocytic leukemia, and it has been confirmed that a BTK inhibitor shows efficacy to some extent in a patient having these cancers (Scand. J. Immunol. 2013; 78(2):130-139). In addition, it has been known that even in the histiocytic lymphoma cell line, the expression of BTK has been confirmed (J. Immunol. 1994; 152(2):557-565).

These results show that in cancers in which BTK is involved, including leukemia and lymphoma, for example, cancers in which BTK is overexpressed or activated, or cancers in which the B cell receptor signal is activated, BTK is involved in proliferation of these cancers, and that a BTK inhibitor may be a promising agent for treating these cancers.

JAK3 (Janus kinase 3) is a non-receptor type tyrosine kinase which belongs to the JAK family of kinases, and is a protein which has a FERM domain, a Src homology 2 domain, a pseudokinase domain, and a kinase domain (Genome Biol. 2004; 5:253). JAK3 is mainly expressed in hematopoietic cells and interacts with a cytokine receptor to deliver the signal to downstream STAT (Acta. Biochim. Biophys. Sin. 2012; 44(3):187-196).

Hitherto, it has been reported that JAK3 is activation-mutated in acute megakaryocytic leukemia, natural killer/T cell lymphoma, cutaneous T cell lymphoma, and T cell acute lymphoblastic leukemia, and it has been suggested that JAK3 is involved in the progression of these cancers (Cancer Cell. 2006; 10:65-75, Cancer Discov. 2012; 2(7):591-597, Blood. 2009; 113:2746-2754, and Leukemia. 2012; 26:2144-2146). In addition, it has been reported that a JAK inhibitor inhibits proliferation of cells of acute megakaryocytic leukemia cells, natural killer/T cell lymphoma cells, and anaplastic large cell lymphoma cells (Cancer Cell. 2006; 10:65-75, Cancer Discov. 2012; 2(7):591-597, and Leukemia. 2014; 28:941-944).

These results show that in cancers in which JAK3 is involved, including leukemia and lymphoma, for example, cancers in which JAK3 is activation-mutated or activated, JAK3 is involved in proliferation of these cancers, and that a JAK3 inhibitor may be a promising agent for treating these cancers.

ITK (IL2 inducible T cell kinase) is a non-receptor type tyrosine kinase which belongs to the Tec family of kinases, and is a protein which has a pleckstrin homology domain, a Tec homology domain, a Src homology 2 domain, a Src homology 3 domain, and a kinase domain. ITK is mainly expressed in T cells and mast cells as well, and is responsible for adjusting the T cell receptor signaling (Curr. Top. Med. Chem. 2009; 9(8):690-703, and Mol. Pharmacol. 2012; 82:938-947).

Hitherto, it has been reported that ITK is expressed in T cell tumors including cutaneous T cell lymphoma. Also, it has been reported that an ITK inhibitor inhibits proliferation of cells of T cell acute lymphoblastic leukemia cells in which ITK is expressed (Mol. Pharmacol. 2012; 82:938-947).

These results show that in cancers in which ITK is involved, including leukemia and lymphoma, for example, cancers in which ITK is activated, ITK is involved in proliferation of these cancers, and that an ITK inhibitor may be a promising agent for treating these cancers.

It has been known that 5-{[(3R)-1-acryloylpyrrolidin-3-yl]oxy}-6-ethyl-3-({4-[4-(4-methylpiperazin-1-yl)piperidin-1-yl]phenyl}amino)pyrazine-2-carboxamide (hereinafter, may be referred to as "compound A") or a pharmaceutically acceptable salt thereof is useful as an active ingredient of a pharmaceutical composition for treating EGFR T790M mutation positive cancer (Patent Document 1).

In Patent Document 1, the compound A or a pharmaceutically acceptable salt thereof is disclosed as the free base in Example 54 and as the monomethanesulfonate thereof in Example 261, and the inhibiting effect against the EGFR mutation kinase is confirmed.

### Related Art

### Patent Document

Patent Document 1: WO 2013/108754

### Disclosure of Invention

### Problems to Be Solved by the Invention

There is provided a pharmaceutical composition for treating cancer, in one aspect, a pharmaceutical composition for treating cancer in which BTK is overexpressed or activated, in another aspect, a pharmaceutical composition for treating cancer in which JAK3 is activation-mutated or activated, and in still another aspect, a pharmaceutical composition for treating cancer in which ITK is activated.

### Means for Solving the Problems

The present inventors have conducted extensive studies for the purpose of preparing a pharmaceutical composition for treating cancer, and as a result, they have found that a pharmaceutical composition comprising 5-{[(3R)-1-acryloylpyrrolidin-3-yl]oxy}-6-ethyl-3-({4- [4-(4-methylpiperazin-1-yl)piperidin-1-yl]phenyl}amino)pyrazine-2-carboxamide or a pharmaceutically acceptable salt thereof as an active ingredient is useful as a pharmaceutical composition for treating cancer in which one or more kinases of BTK, JAK3, and ITK is involved, thereby completing the present invention.

That is, the present invention is:
1. A pharmaceutical composition for treating cancer in which one or more kinases of BTK, JAK3, and ITK is involved, comprising 5-{[(3R)-1-acryloylpyrrolidin-3-yl]oxy}-6-ethyl-3-({4-[4-(4-methylpiperazin-1-yl)piperidin-1-yl]phenyl}amino)pyrazine-2-carboxamide or a pharmaceutically acceptable salt thereof as an active ingredient.
2. The pharmaceutical composition described in 1, wherein the cancer in which one or more kinases of BTK, JAK3, and ITK is involved is cancer in which BTK is involved.
3. The pharmaceutical composition described in 2, wherein the cancer in which BTK is involved is cancer in which BTK is overexpressed or activated.
4. The pharmaceutical composition described in 2, wherein the cancer in which BTK is involved is cancer in which a B cell receptor signal is activated.
5. The pharmaceutical composition described in 2, wherein the cancer in which BTK is involved is cancer selected from the group consisting of follicular lymphoma, chronic lymphocytic leukemia, small lymphocytic lymphoma, multiple myeloma, mantle cell lymphoma, diffuse large B-cell lymphoma, B-cell prolymphocytic leukemia, acute lymphocytic leukemia, histiocytic lymphoma, and acute myeloid leukemia.
6. The pharmaceutical composition described in 5, wherein the cancer in which BTK is involved is diffuse large B-cell lymphoma, mantle cell lymphoma, acute myeloid leukemia and/or histiocytic lymphoma.
7. The pharmaceutical composition described in 1, wherein the cancer in which one or more kinases of BTK, JAK3, and ITK is involved is cancer in which JAK3 is involved.
8. The pharmaceutical composition described in 7, wherein the cancer in which JAK3 is involved is cancer in which JAK3 is activation-mutated or activated.
9. The pharmaceutical composition described in 7, wherein the cancer in which JAK3 is involved is acute megakaryocytic leukemia and/or anaplastic large cell lymphoma.
10. The pharmaceutical composition described in 1, wherein the cancer in which one or more kinases of BTK, JAK3, and ITK is involved is cancer in which ITK is involved.
11. The pharmaceutical composition described in 10, wherein the cancer in which ITK is involved is cancer in which ITK is activated.
12. The pharmaceutical composition described in 10, wherein the cancer in which ITK is involved is acute T cell lymphoma and/or lymphoblastic leukemia.
13. The pharmaceutical composition described in 1, wherein the cancer in which one or more kinases of BTK, JAK3, and ITK is involved is cancer in which BTK and JAK3 are involved.
14. The pharmaceutical composition described in 1, wherein the cancer in which one or more kinases of BTK, JAK3, and ITK is involved is cancer in which BTK and ITK are involved.
15. The pharmaceutical composition described in 1, wherein the cancer in which one or more kinases of BTK, JAK3, and ITK is involved is cancer in which JAK3 and ITK are involved.
16. The pharmaceutical composition according to claim 1, wherein the cancer in which one or more kinases of BTK, JAK3, and ITK is involved is cancer in which BTK, JAK3 and ITK are involved.
17. The pharmaceutical composition described in any of 1 to 16, wherein 5-{[(3R)-1-acryloylpyrrolidin-3-yl]oxy}-6-ethyl-3-({4-[4-(4-methylpiperazin-1-yl)piperidin-1-yl]phenyl}amino)pyrazine-2-carboxamide or a pharmaceutically acceptable salt thereof is 5- {[(3R)-1-acryloylpyrrolidin-3-yl]oxy}-6-ethyl-3-({4-[4-(4-methylpiperazin-1-yl)piperidin-1-yl]phenyl}amino)pyrazine-2-carboxamide monomethanesulfonate.
18. Use of the compound A or a pharmaceutically acceptable salt thereof for the manufacture of a pharmaceutical composition for treating cancer in which one or more kinases of BTK, JAK3, and ITK is involved.
19. Use of the compound A or a pharmaceutically acceptable salt thereof for treating cancer in which one or more kinases of BTK, JAK3, and ITK is involved.
20. The compound A or a pharmaceutically acceptable salt thereof for treating cancer in which one or more kinases of BTK, JAK3, and ITK is involved.
21. A method for treating cancer in which one or more kinases of BTK, JAK3, and ITK is involved, comprising administering an effective amount of the compound A or a pharmaceutically acceptable salt thereof to a subject.

In addition, the present invention includes an agent for treating cancer in which one or more kinases of BTK, JAK3, and ITK is involved, comprising the compound A or a pharmaceutically acceptable salt thereof; in one aspect, an agent for treating cancer in which BTK is involved, where the cancer in which one or more kinases of BTK, JAK3, and ITK is involved is the cancer in which BTK is involved; in another aspect, an agent for treating cancer in which BTK is overexpressed or activated; in still another aspect, an agent for treating cancer in which a B cell receptor signal is activated; in still another aspect, an agent for treating cancer in which JAK3 is involved, where the cancer in which one or more kinases of BTK, JAK3, and ITK is involved is the cancer in which JAK3 is involved; in still another aspect, an agent for treating cancer in which JAK3 is activation-mutated or activated; in still another aspect, an agent for treating cancer in which ITK is involved, where the cancer in which one or more kinases of BTK, JAK3, and ITK is involved is the cancer in which ITK is involved; and in still another aspect, an agent for treating cancer in which ITK is activated.

In addition, the present invention relates to use of the compound A or a pharmaceutically acceptable salt thereof for the manufacture of a pharmaceutical composition for treating cancer in which one or more kinases of BTK, JAK3, and ITK is involved, in one aspect, use of the compound A or a pharmaceutically acceptable salt thereof for the manufacture of a pharmaceutical composition for treating cancer in which BTK is involved, in another aspect, use of the compound A or a pharmaceutically acceptable salt thereof for the manufacture of a pharmaceutical composition for treating cancer in which BTK is overexpressed or activated, in still another aspect, use of the compound A or a pharmaceutically acceptable salt thereof for the manufacture of a pharmaceutical composition for treating cancer in which a B cell receptor signal is activated, in still another aspect, use of the compound A or a pharmaceutically acceptable salt thereof for the manufacture of a pharmaceutical composition for treating cancer in which JAK3 is involved, in still another aspect, use of the compound A or a pharmaceutically acceptable salt thereof for the manufacture of a pharmaceutical composition for treating cancer in which JAK3 is activation-mutated or activated, in still another aspect, use of the compound A or a pharmaceutically acceptable salt thereof for the manufacture of a pharmaceutical composition for treating cancer in which ITK is involved, and in still another aspect, use of the compound A or a pharmaceutically acceptable salt thereof for the manufacture of a pharmaceutical composition for treating cancer in which ITK is involved; use of the compound A or a pharmaceutically acceptable salt thereof for treating cancer in which one or more kinases of BTK, JAK3, and ITK is involved, in one aspect, use of the compound A or a pharmaceutically acceptable salt thereof for treating cancer in which BTK is involved, in another aspect, use of the compound A or a pharmaceutically acceptable salt thereof for treating cancer in which BTK is overexpressed or activated, in still another aspect, use of the compound A or a pharmaceutically acceptable salt thereof for treating cancer in which a B cell receptor signal is activated, in still another aspect, use of the compound A or a pharmaceutically acceptable salt thereof for treating cancer in which JAK3 is involved, in still another aspect, use of the compound A or a pharmaceutically acceptable salt thereof for treating cancer in which JAK3 is activation-mutated or activated, in still another aspect, use of the compound A or a pharmaceutically acceptable salt thereof for treating cancer in which ITK is involved, and in still another aspect, use of the compound A or a pharmaceutically acceptable salt thereof for treating cancer in which ITK is activated; the compound A or a pharmaceutically acceptable salt thereof for treating cancer in which one or more kinases of BTK, JAK3, and ITK is involved, in one aspect, the compound A or a pharmaceutically acceptable salt thereof for treating cancer in which BTK is involved, in another aspect, the compound A or a pharmaceutically acceptable salt thereof for treating cancer in which BTK is overexpressed or activated, in still another aspect, the compound A or a pharmaceutically acceptable salt thereof for treating cancer in which a B cell receptor signal is activated, in still another aspect, the compound A or a pharmaceutically acceptable salt thereof for treating cancer in which JAK3 is involved, in still another aspect, the compound A or a pharmaceutically acceptable salt thereof for treating cancer in which JAK3 is activation-mutated or activated, in still another aspect, the compound A or a pharmaceutically acceptable salt thereof for treating cancer in which ITK is involved, and in still another aspect, the compound A or a pharmaceutically acceptable salt thereof for treating cancer in which ITK is activated; and a method for treating cancer in which one or more kinases of BTK, JAK3, and ITK is involved, comprising administering an effective amount of the compound A or a pharmaceutically acceptable salt thereof to a subject, in one aspect, a method for treating cancer in which BTK is involved, comprising administering an effective amount of the compound A or a pharmaceutically acceptable salt thereof to a subject, in another aspect, a method for treating cancer in which BTK is overexpressed or activated, comprising administering an effective amount of the compound A or a pharmaceutically acceptable salt thereof to a subject, in still another aspect, a method for treating cancer in which a B cell receptor signal is activated, comprising administering an effective amount of the compound A or a pharmaceutically acceptable salt thereof to a subject, in still another aspect, a method for treating cancer in which JAK3 is involved, comprising administering an effective amount of the compound A or a pharmaceutically acceptable salt thereof to a subject, in still another aspect, a method for treating cancer in which JAK3 is activation-mutated or activated, comprising administering an effective amount of the compound A or a pharmaceutically acceptable salt thereof to a subject, in still another aspect a method for treating cancer in which ITK is involved, comprising administering an effective amount of the compound A or a pharmaceutically acceptable salt thereof to a subject, and in still another aspect, a method for treating cancer in which ITK is activated, comprising administering an effective amount of the compound A or a pharmaceutically acceptable salt thereof to a subject. In addition, the "subject" is a human or another animal which needs the treatment, and, in one aspect, is a human which needs the treatment.

### Effects of the Invention

The compound A or a pharmaceutically acceptable salt thereof, which is an active ingredient of the pharmaceutical composition of the present invention, has BTK, JAK3, and ITK inhibiting effects and can be used as an active ingredient for a pharmaceutical composition for treating cancer in which one or more kinases of BTK, JAK3, and ITK is involved, in one aspect, a pharmaceutical composition for treating cancer in which BTK is involved, cancer in which BTK is overexpressed or activated, cancer in which a B cell receptor signal is activated, cancer in which JAK3 is involved, cancer in which JAK3 is activation-mutated or activated, cancer in which ITK is involved, and/or cancer in which ITK is activated, in another aspect, a pharmaceutical composition for treating cancer in which BTK is overexpressed or activated, instill another aspect, a pharmaceutical composition for treating cancer in which a B cell receptor signal is activated, in still another aspect, a pharmaceutical composition for treating cancer in which JAK3 is activation-mutated or activated, and in still another aspect, a pharmaceutical composition for treating cancer in which ITK is activated.

### Embodiments for Carrying Out the Invention

Hereinafter, the present invention will be described in detail.

As described above, a chemical name of the compound A is 5-{[(3R)-1-acryloylpyrrolidin-3-yl]oxy}-6-ethyl-3-({4-[4-(4-methylpiperazin-1-yl)piperidin-1-yl]phenyl}amino)pyrazine-2-carboxamide, and a chemical structure thereof is shown as follows.

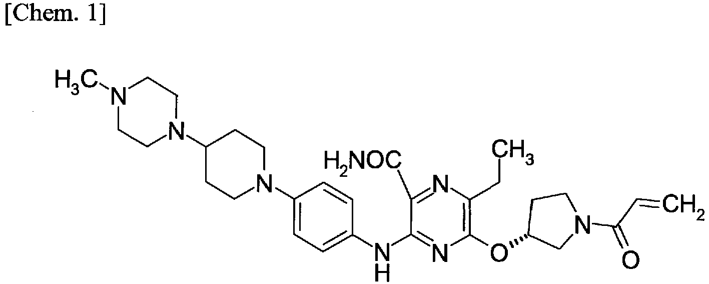

The cancer in which BTK is involved means cancer in which BTK is one of the causes of the cancer, and examples thereof include cancer in which BTK is overexpressed or activated, and cancer in which a B cell receptor signal is activated. Specific examples thereof include follicular lymphoma, chronic lymphocytic leukemia, small lymphocytic lymphoma, multiple myeloma, mantle cell lymphoma, diffuse large B-cell lymphoma, B-cell prolymphocytic leukemia, acute lymphocytic leukemia, histiocytic lymphoma, and acute myeloid leukemia; and, in particular, diffuse large B-cell lymphoma, mantle cell lymphoma, acute myeloid leukemia, and histiocytic lymphoma.

The cancer in which JAK3 is involved means cancer in which JAK3 is one of the causes of the cancer, and examples thereof include cancer in which JAK3 is overexpressed or activated, and cancer in which JAK3 is activation-mutated, for example, cancer having mutation from proline at position 132 in JAK3 defined as GenBank Accession Number: NM_000215.3, to threonine, mutation from alanine at position 572 to valine, and mutation from valine at position 722 to isoleucine (Cancer Cell. 2006;10:65-75), and include acute megakaryocytic leukemia and anaplastic large cell lymphoma.

The cancer in which ITK is involved means cancer in which ITK is one of the causes of the cancer, and examples thereof include cancer in which ITK is overexpressed or activated, and includes acute T cell lymphoma and lymphoblastic leukemia.

In addition, the "cancer" in the present specification indicates hematologic cancer.

One aspect of the present invention is shown below.
(1-1) A pharmaceutical composition for treating cancer in which BTK is involved, comprising the compound A or a pharmaceutically acceptable salt thereof. In one aspect, a pharmaceutical composition for treating cancer in which BTK is overexpressed or activated, comprising the compound A or a pharmaceutically acceptable salt thereof. In another aspect, a pharmaceutical composition for treating cancer in which a B cell receptor signal is activated, comprising the compound A or a pharmaceutically acceptable salt thereof.
(1-2) Use of the compound A or a pharmaceutically acceptable salt thereof for the manufacture of a pharmaceutical composition for treating cancer in which BTK is involved. In one aspect, use of the compound A or a pharmaceutically acceptable salt thereof for the manufacture of a pharmaceutical composition for treating cancer in which BTK is overexpressed or activated. In another aspect, use of the compound A or a pharmaceutically acceptable salt thereof for the manufacture of a pharmaceutical composition for treating cancer in which a B cell receptor signal is activated.
(1-3) Use of the compound A or a pharmaceutically acceptable salt thereof for treating cancer in which BTK is involved. In one aspect, use of the compound A or a pharmaceutically acceptable salt thereof for treating cancer in which BTK is overexpressed or activated. In another aspect, use of the compound A or a pharmaceutically acceptable salt thereof for treating cancer in which a B cell receptor signal is activated.
(1-4) The compound A or a pharmaceutically acceptable salt thereof for treating cancer in which BTK is involved. In one aspect, the compound A or a pharmaceutically acceptable salt thereof for treating cancer in which BTK is overexpressed or activated. In another aspect, the compound A or a pharmaceutically acceptable salt thereof for treating cancer in which a B cell receptor signal is activated.
(1-5) A method for treating cancer in which BTK is involved, comprising administering an effective amount of the compound A or a pharmaceutically acceptable salt thereof to a subject. In one aspect, a method for treating cancer in which BTK is overexpressed or activated, comprising administering an effective amount of the compound A or a pharmaceutically acceptable salt thereof to a subject. In another aspect, a method for treating cancer in which a B cell receptor signal is activated, comprising administering an effective amount of the compound A or a pharmaceutically acceptable salt thereof to a subject.
(2-1) A pharmaceutical composition for treating cancer in which BTK is involved, comprising the compound A monomethanesulfonate. In one aspect, a pharmaceutical composition for treating cancer in which BTK is overexpressed or activated, comprising the compound A monomethanesulfonate. In another aspect, a pharmaceutical composition for treating cancer in which a B cell receptor signal is activated, comprising the compound A monomethanesulfonate.
(2-2) Use of the compound A monomethanesulfonate for the manufacture of a pharmaceutical composition for treating cancer in which BTK is involved. In one aspect, use of the compound A monomethanesulfonate for the manufacture of a pharmaceutical composition for treating cancer in which BTK is overexpressed or activated. In another aspect, use of the compound A monomethanesulfonate for the manufacture of a pharmaceutical composition for treating cancer in which a B cell receptor signal is activated.
(2-3) Use of the compound A monomethanesulfonate for treating cancer in which BTK is involved. In one aspect, use of the compound A monomethanesulfonate for treating cancer in which BTK is overexpressed or activated. In another aspect, use of the compound A monomethanesulfonate for treating cancer in which a B cell receptor signal is activated.
(2-4) The compound A monomethanesulfonate for treating cancer in which BTK is involved. In one aspect, the compound A monomethanesulfonate for treating cancer in which BTK is overexpressed or activated. In another aspect, the compound A monomethanesulfonate for treating cancer in which a B cell receptor signal is activated.
(2-5) A method for treating cancer in which BTK is involved, comprising administering an effective amount of the compound A monomethanesulfonate to a subject. In one aspect, a method for treating cancer in which BTK is overexpressed or activated, comprising administering an effective amount of the compound A monomethanesulfonate to a subject. In another aspect, a method for treating cancer in which a B cell receptor signal is activated, comprising administering an effective amount of the compound A monomethanesulfonate to a subject.
(3) The pharmaceutical composition disclosed (1-1) or (2-1); the use disclosed in (1-2) or (2-2); the use disclosed in (1-3) or (2-3); the compound A disclosed in (1-4) or the compound A monomethanesulfonate disclosed in (2-4); or the treatment method disclosed in (1-5) or (2-5), where the cancer in which BTK is involved, the cancer in which BTK is overexpressed or activated, or the cancer in which a B cell receptor signal is activated is follicular lymphoma, chronic lymphocytic leukemia, small lymphocytic lymphoma, multiple myeloma, mantle cell lymphoma, diffuse large B-cell lymphoma, B-cell prolymphocytic leukemia, acute lymphocytic leukemia, histiocytic lymphoma, or acute myeloid leukemia.
   In another aspect, the pharmaceutical composition disclosed in (1-1) or (2-1); the use disclosed in (1-2) or (2-2); the use disclosed in (1-3) or (2-3); the compound A disclosed in (1-4) or the compound A monomethanesulfonate disclosed in (2-4); or the treatment method disclosed in (1-5) or (2-5), where the cancer in which BTK is involved, the cancer in which BTK is overexpressed or activated, or the cancer in which a B cell receptor signal is activated is diffuse large B-cell lymphoma, mantle cell lymphoma, acute myeloid leukemia, or histiocytic lymphoma.
   In still another aspect, the pharmaceutical composition disclosed in (1-1) or (2-1); the use disclosed in (1-2) or (2-2); the use disclosed in (1-3) or (2-3); the compound A disclosed in (1-4) or the compound A monomethanesulfonate disclosed in (2-4); or the treatment method disclosed in (1-5) or (2-5), where the cancer in which BTK is involved, the cancer in which BTK is overexpressed or activated, or the cancer in which a B cell receptor signal is activated is diffuse large B-cell lymphoma.
   In still another aspect, the pharmaceutical composition disclosed in (1-1) or (2-1); the use disclosed in (1-2) or (2-2); the use disclosed in (1-3) or (2-3); the compound A disclosed in (1-4) or the compound A monomethanesulfonate disclosed in (2-4); or the treatment method disclosed in (1-5) or (2-5), where the cancer in which BTK is involved, the cancer in which BTK is overexpressed or activated, or the cancer in which a B cell receptor signal is activated is mantle cell lymphoma.
   In still another aspect, the pharmaceutical composition disclosed in (1-1) or (2-1); the use disclosed in (1-2) or (2-2); the use disclosed in (1-3) or (2-3); the compound A disclosed in (1-4) or the compound A monomethanesulfonate disclosed in (2-4); or the treatment method disclosed in (1-5) or (2-5), where the cancer in which BTK is involved, the cancer in which BTK is overexpressed or activated, or the cancer in which a B cell receptor signal is activated is acute myeloid leukemia.
   In still another aspect, the pharmaceutical composition disclosed in (1-1) or (2-1); the use disclosed in (1-2) or (2-2); the use disclosed in (1-3) or (2-3); the compound A disclosed in (1-4) or the compound A monomethanesulfonate disclosed in (2-4); or the treatment method disclosed in (1-5) or (2-5), where the cancer in which BTK is involved, the cancer in which BTK is overexpressed or activated, or the cancer in which a B cell receptor signal is activated is histiocytic lymphoma.
(4-1) A pharmaceutical composition for treating cancer in which JAK3 is involved, comprising the compound A or a pharmaceutically acceptable salt thereof. In one aspect, a pharmaceutical composition for treating cancer in which JAK3 is activation-mutated or activated, comprising the compound A or a pharmaceutically acceptable salt thereof.
(4-2) Use of the compound A or a pharmaceutically acceptable salt thereof for the manufacture of a pharmaceutical composition for treating cancer in which JAK3 is involved. In one aspect, use of the compound A or a pharmaceutically acceptable salt thereof for the manufacture of a pharmaceutical composition for treating cancer in which JAK3 is activation-mutated or activated.
(4-3) Use of the compound A or a pharmaceutically acceptable salt thereof for treating cancer in which JAK3 is involved. In one aspect, use of the compound A or a pharmaceutically acceptable salt thereof for treating cancer in which JAK3 is activation-mutated or activated.
(4-4) The compound A or a pharmaceutically acceptable salt thereof for treating cancer in which JAK3 is involved. In one aspect, the compound A or a pharmaceutically acceptable salt thereof for treating cancer in which JAK3 is activation-mutated or activated.
(4-5) A method for treating cancer in which JAK3 is involved, comprising administering an effective amount of the compound A or a pharmaceutically acceptable salt thereof to a subject. In one aspect, a method for treating cancer in which JAK3 is activation-mutated or activated, comprising administering an effective amount of the compound A or a pharmaceutically acceptable salt thereof to a subject.
(5-1) A pharmaceutical composition for treating cancer in which JAK3 is involved, comprising the compound A monomethanesulfonate. In one aspect, a pharmaceutical composition for treating cancer in which JAK3 is activation-mutated or activated, comprising the compound A monomethanesulfonate.
(5-2) Use of the compound A monomethanesulfonate for the manufacture of a pharmaceutical composition for treating cancer in which JAK3 is involved. In one aspect, use of the compound A monomethanesulfonate for the manufacture of a pharmaceutical composition for treating cancer in which JAK3 is activation-mutated or activated.
(5-3) Use of the compound A monomethanesulfonate for treating cancer in which JAK3 is involved. In one aspect, use of the compound A monomethanesulfonate for treating cancer in which JAK3 is activation-mutated or activated.
(5-4) The compound A monomethanesulfonate for treating cancer in which JAK3 is involved. In one aspect, the compound A monomethanesulfonate for treating cancer in which JAK3 is activation-mutated or activated.
(5-5) A method for treating cancer in which JAK3 is involved, comprising administering an effective amount of the compound A monomethanesulfonate to a subject. In one aspect, a method for treating cancer in which JAK3 is activation-mutated or activated, comprising administering an effective amount of the compound A monomethanesulfonate to a subject.
(6) The pharmaceutical composition disclosed in (4-1) or (5-1); the use disclosed in (4-2) or (5-2); the use disclosed in (4-3) or (5-3); the compound A disclosed in (4-4) or the compound A monomethanesulfonate disclosed in (5-4); or the treatment method disclosed in (4-5) or (5-5), where the cancer in which JAK3 is involved, or the cancer in which JAK3 is activation-mutated or activated is acute megakaryocytic leukemia or anaplastic large cell lymphoma.
   In another aspect, the pharmaceutical composition disclosed in (4-1) or (5-1); the use disclosed in (4-2) or (5-2); the use disclosed in (4-3) or (5-3); the compound A disclosed in (4-4) or the compound A monomethanesulfonate disclosed in (5-4); or the treatment method disclosed in (4-5) or (5-5), where the cancer in which JAK3 is involved, or the cancer in which JAK3 is activation-mutated or activated is acute megakaryocytic leukemia.
   In still another aspect, the pharmaceutical composition disclosed in (4-1) or (5-1); the use disclosed in (4-2) or (5-2); the use disclosed in (4-3) or (5-3); the compound A disclosed in (4-4) or the compound A monomethanesulfonate disclosed in (5-4); or the treatment method disclosed in (4-5) or (5-5), where the cancer in which JAK3 is involved, or the cancer in which JAK3 is activation-mutated or activated is anaplastic large cell lymphoma.
(7-1) A pharmaceutical composition for treating cancer in which ITK is involved, comprising the compound A or a pharmaceutically acceptable salt thereof. In one aspect, a pharmaceutical composition for treating cancer in which ITK is activated, comprising the compound A or a pharmaceutically acceptable salt thereof.
(7-2) Use of the compound A or a pharmaceutically acceptable salt thereof for the manufacture of a pharmaceutical composition for treating cancer in which ITK is involved. In one aspect, use of the compound A or a pharmaceutically acceptable salt thereof for the manufacture of a pharmaceutical composition for treating cancer in which ITK is activated.
(7-3) Use of the compound A or a pharmaceutically acceptable salt thereof for treating cancer in which ITK is involved. In one aspect, use of the compound A or a pharmaceutically acceptable salt thereof for treating cancer in which ITK is activated.
(7-4) The compound A or a pharmaceutically acceptable salt thereof for treating cancer in which ITK is involved. In one aspect, the compound A or a pharmaceutically acceptable salt thereof for treating cancer in which ITK is activated.
(7-5) A method for treating cancer in which ITK is involved, comprising administering an effective amount of the compound A or a pharmaceutically acceptable salt thereof to a subject. In one aspect, a method for treating cancer in which ITK is activated, comprising administering an effective amount of the compound A or a pharmaceutically acceptable salt thereof to a subject.
(8-1) A pharmaceutical composition for treating cancer in which ITK is involved, comprising the compound A monomethanesulfonate. In one aspect, a pharmaceutical composition for treating cancer in which ITK is activated, comprising the compound A monomethanesulfonate.
(8-2) Use of the compound A monomethanesulfonate for the manufacture of a pharmaceutical composition for treating cancer in which ITK is involved. In one aspect, use of the compound A monomethanesulfonate for the manufacture of a pharmaceutical composition for treating cancer in which ITK is activated.
(8-3) Use of the compound A monomethanesulfonate for treating cancer in which ITK is involved. In one aspect, use of the compound A monomethanesulfonate for treating cancer in which ITK is activated.
(8-4) The compound A monomethanesulfonate for treating cancer in which ITK is involved. In one aspect, the compound A monomethanesulfonate for treating cancer in which ITK is activated.
(8-5) A method for treating cancer in which ITK is involved, comprising administering an effective amount of the compound A monomethanesulfonate to a subject. In one aspect, a method for treating cancer in cancer in which ITK is activated, comprising administering an effective amount of the compound A monomethanesulfonate to a subject.
(9) The pharmaceutical composition disclosed in (7-1) or (8-1); the use disclosed in (7-2) or (8-2); the use disclosed in (7-3) or (8-3); the compound A disclosed in (7-4) or the compound A monomethanesulfonate disclosed in (8-4); or the treatment method disclosed in (7-5) or (8-5), where the cancer in which ITK is involved or the cancer in which ITK is activated is acute T cell lymphoma or lymphoblastic leukemia.
   In another aspect, the pharmaceutical composition disclosed in (7-1) or (8-1); the use disclosed in (7-2) or (8-2); the use disclosed in (7-3) or (8-3); the compound A disclosed in (7-4) or the compound A monomethanesulfonate disclosed in (8-4); or the treatment method disclosed in (7-5) or (8-5), where the cancer in which ITK is involved or the cancer in which ITK is activated is acute T cell lymphoma.
   In still another aspect, the pharmaceutical composition disclosed in (7-1) or (8-1); the use disclosed in (7-2) or (8-2); the use disclosed in (7-3) or (8-3); the compound A disclosed in (7-4) or the compound A monomethanesulfonate disclosed in (8-4); or the treatment method disclosed in (7-5) or (8-5), where the cancer in which ITK is involved or the cancer in which ITK is activated is lymphoblastic leukemia.
(10-1) A pharmaceutical composition for treating cancer in which BTK and JAK3 are involved, comprising the compound A or a pharmaceutically acceptable salt thereof. In one aspect, a pharmaceutical composition for treating cancer in which BTK is overexpressed or activated and cancer in which JAK3 is activation-mutated or activated, comprising the compound A or a pharmaceutically acceptable salt thereof. In another aspect, a pharmaceutical composition for treating cancer in which a B cell receptor signal is activated and cancer in which JAK3 is activation-mutated or activated, comprising the compound A or a pharmaceutically acceptable salt thereof.
(10-2) Use of the compound A or a pharmaceutically acceptable salt thereof for the manufacture of a pharmaceutical composition for treating cancer in which BTK and JAK3 are involved. In one aspect, use of the compound A or a pharmaceutically acceptable salt thereof for the manufacture of a pharmaceutical composition for treating cancer in which BTK is overexpressed or activated and cancer in which JAK3 is activation-mutated or activated. In another aspect, use of the compound A or a pharmaceutically acceptable salt thereof for the manufacture of a pharmaceutical composition for treating cancer in which a B cell receptor signal is activated and cancer in which JAK3 is activation-mutated or activated.
(10-3) Use of the compound A or a pharmaceutically acceptable salt thereof for treating cancer in which BTK and JAK3 are involved. In one aspect, use of the compound A or a pharmaceutically acceptable salt thereof for treating cancer in which BTK is overexpressed or activated and cancer in which JAK3 is activation-mutated or activated. In another aspect, use of the compound A or a pharmaceutically acceptable salt thereof for treating cancer in which a B cell receptor signal is activated and cancer in which JAK3 is activation-mutated or activated.
(10-4) The compound A or a pharmaceutically acceptable salt thereof for treating cancer in which BTK and JAK3 are involved. In one aspect, the compound A or a pharmaceutically acceptable salt thereof for treating cancer in which BTK is overexpressed or activated and cancer in which JAK3 is activation-mutated or activated. In another aspect, the compound A or a pharmaceutically acceptable salt thereof for treating cancer in which a B cell receptor signal is activated and cancer in which JAK3 is activation-mutated or activated.
(10-5) A method for treating cancer in which BTK and JAK3 are involved, comprising administering an effective amount of the compound A or a pharmaceutically acceptable salt thereof. In one aspect, a method for treating cancer in which BTK is overexpressed or activated and cancer in which JAK3 is activation-mutated or activated, comprising administering an effective amount of the compound A or a pharmaceutically acceptable salt thereof. In another aspect, a method for treating cancer in which a B cell receptor signal is activated and cancer in which JAK3 is activation-mutated or activated, comprising administering an effective amount of the compound A or a pharmaceutically acceptable salt thereof.
(11-1) A pharmaceutical composition for treating cancer in which BTK and JAK3 are involved, comprising the compound A monomethanesulfonate. In one aspect, a pharmaceutical composition for treating cancer in which BTK is overexpressed or activated and cancer in which JAK3 is activation-mutated or activated, comprising the compound A monomethanesulfonate. In another aspect, a pharmaceutical composition for treating cancer in which a B cell receptor signal is activated and cancer in which JAK3 is activation-mutated or activated, comprising the compound A monomethanesulfonate.
(11-2) Use of the compound A monomethanesulfonate for the manufacture of a pharmaceutical composition for treating cancer in which BTK and JAK3 are involved. In one aspect, use of the compound A monomethanesulfonate for the manufacture of a pharmaceutical composition for treating cancer in which BTK is overexpressed or activated and cancer in which JAK3 is activation-mutated or activated. In another aspect, use of the compound A monomethanesulfonate for the manufacture of a pharmaceutical composition for treating cancer in which a B cell receptor signal is activated and cancer in which JAK3 is activation-mutated or activated.
(11-3) Use of the compound A monomethanesulfonate for treating cancer in which BTK and JAK3 are involved. In one aspect, use of the compound A monomethanesulfonate for treating cancer in which BTK is overexpressed or activated and cancer in which JAK3 is activation-mutated or activated. In another aspect, use of the compound A monomethanesulfonate for treating cancer in which a B cell receptor signal is activated and cancer in which JAK3 is activation-mutated or activated.
(11-4) The compound A monomethanesulfonate for treating cancer in which BTK and JAK3 are involved. In one aspect, the compound A monomethanesulfonate for treating cancer in which BTK is overexpressed or activated and cancer in which JAK3 is activation-mutated or activated. In another aspect, the compound A monomethanesulfonate for treating cancer in which a B cell receptor signal is activated and cancer in which JAK3 is activation-mutated or activated.
(11-5) A method for treating cancer in which BTK and JAK3 are involved, comprising administering an effective amount of the compound A monomethanesulfonate to a subject. In one aspect, a treatment method for cancer in which BTK is overexpressed or activated and cancer in which JAK3 is activation-mutated or activated, comprising administering an effective amount of the compound A monomethanesulfonate to a subject. In another aspect, a method for treating cancer in which a B cell receptor signal is activated and cancer in which JAK3 is activation-mutated or activated, comprising administering an effective amount of the compound A monomethanesulfonate to a subject.
(12) The pharmaceutical composition disclosed in (10-1) or (11-1); the use disclosed in (10-2) or (11-2); the use disclosed in (10-3) or (11-3); the compound A disclosed in (10-4) or the compound A monomethanesulfonate disclosed in (11-4); or the treatment method disclosed in (10-5) or (11-5), where the cancer in which BTK is involved, the cancer in which BTK is overexpressed or activated, or the cancer in which a B cell receptor signal is activated is follicular lymphoma, chronic lymphocytic leukemia, small lymphocytic lymphoma, multiple myeloma, mantle cell lymphoma, diffuse large B-cell lymphoma, B-cell prolymphocytic leukemia, acute lymphocytic leukemia, histiocytic lymphoma, or acute myeloid leukemia, and the cancer in which JAK3 is involved or the cancer in which JAK3 is activation-mutated or activated is acute megakaryocytic leukemia or anaplastic large cell lymphoma.
   In another aspect, the pharmaceutical composition disclosed in (10-1) or (11-1); the use disclosed in (10-2) or (11-2); the use disclosed in (10-3) or (11-3); the compound A disclosed in (10-4) or the compound A monomethanesulfonate disclosed in (11-4); or the treatment method disclosed in (10-5) or (11-5), where the cancer in which BTK is involved, the cancer in which BTK is overexpressed or activated, or the cancer in which a B cell receptor signal is activated is diffuse large B-cell lymphoma, mantle cell lymphoma, acute myeloid leukemia, or histiocytic lymphoma, and the cancer in which JAK3 is involved or the cancer in which JAK3 is activation-mutated or activated is acute megakaryocytic leukemia.
   In still another aspect, the pharmaceutical composition disclosed in (10-1) or (11-1); the use disclosed in (10-2) or (11-2); the use disclosed in (10-3) or (11-3); the compound A disclosed in (10-4) or the compound A monomethanesulfonate disclosed in (11-4); or the treatment method disclosed in (10-5) or (11-5), where the cancer in which BTK is involved, the cancer in which BTK is overexpressed or activated, or cancer in which a B cell receptor signal is activated is diffuse large B-cell lymphoma, mantle cell lymphoma, acute myeloid leukemia, or histiocytic lymphoma, and the cancer in which JAK3 is involved or the cancer in which JAK3 is activation-mutated or activated is anaplastic large cell lymphoma.
   In still another aspect, the pharmaceutical composition disclosed in (10-1) or (11-1); the use disclosed in (10-2) or (11-2); the use disclosed in (10-3) or (11-3); the compound A disclosed in (10-4) or the compound A monomethanesulfonate disclosed in (11-4); or the treatment method disclosed in (10-5) or (11-5), where the cancer in which BTK is involved, the cancer in which BTK is overexpressed or activated, or the cancer in which a B cell receptor signal is activated is diffuse large B-cell lymphoma, and the cancer in which JAK3 is involved or the cancer in which JAK3 is activation-mutated or activated is acute megakaryocytic leukemia.
   In still another aspect, the pharmaceutical composition disclosed in (10-1) or (11-1); the use disclosed in (10-2) or (11-2); the use disclosed in (10-3) or (11-3); the compound A disclosed in (10-4) or the compound A monomethanesulfonate disclosed in (11-4); or the treatment method disclosed in (10-5) or (11-5), where the cancer in which BTK is involved, the cancer in which BTK is overexpressed or activated, or the cancer in which a B cell receptor signal is activated is diffuse large B-cell lymphoma, and the cancer in which JAK3 is involved or the cancer in which JAK3 is activation-mutated or activated is anaplastic large cell lymphoma.
   In still another aspect, the pharmaceutical composition disclosed in (10-1) or (11-1); the use disclosed in (10-2) or (11-2); the use disclosed in (10-3) or (11-3); the compound A disclosed in (10-4) or the compound A monomethanesulfonate disclosed in (11-4); or the treatment method disclosed in (10-5) or (11-5), where the cancer in which BTK is involved, the cancer in which BTK is overexpressed or activated, or the cancer in which a B cell receptor signal is activated is mantle cell lymphoma, and the cancer in which JAK3 is involved or the cancer in which JAK3 is activation-mutated or activated is acute megakaryocytic leukemia.
   In still another aspect, the pharmaceutical composition disclosed in (10-1) or (11-1); the use disclosed in (10-2) or (11-2); the use disclosed in (10-3) or (11-3); the compound A disclosed in (10-4) or the compound A monomethanesulfonate disclosed in (11-4); or the treatment method disclosed in (10-5) or (11-5), where the cancer in which BTK is involved, the cancer in which BTK is overexpressed or activated, or the cancer in which a B cell receptor signal is activated is mantle cell lymphoma, and the cancer in which JAK3 is involved or the cancer in which JAK3 is activation-mutated or activated is anaplastic large cell lymphoma.
   In still another aspect, the pharmaceutical composition disclosed in (10-1) or (11-1); the use disclosed in (10-2) or (11-2); the use disclosed in (10-3) or (11-3); the compound A disclosed in (10-4) or the compound A monomethanesulfonate disclosed in (11-4); or the treatment method disclosed in (10-5) or (11-5), where the cancer in which BTK is involved, the cancer in which BTK is overexpressed or activated, or the cancer in which a B cell receptor signal is activated is acute myeloid leukemia, and the cancer in which JAK3 is involved or the cancer in which JAK3 is activation-mutated or activated is acute megakaryocytic leukemia.
   In still another aspect, the pharmaceutical composition disclosed in (10-1) or (11-1); the use disclosed in (10-2) or (11-2); the use disclosed in (10-3) or (11-3); the compound A disclosed in (10-4) or the compound A monomethanesulfonate disclosed in (11-4); or the treatment method disclosed in (10-5) or (11-5), where the cancer in which BTK is involved, the cancer in which BTK is overexpressed or activated, or the cancer in which a B cell receptor signal is activated is acute myeloid leukemia, and the cancer in which JAK3 is involved or the cancer in which JAK3 is activation-mutated or activated is anaplastic large cell lymphoma.
   In still another aspect, the pharmaceutical composition disclosed in (10-1) or (11-1); the use disclosed in (10-2) or (11-2); the use disclosed in (10-3) or (11-3); the compound A disclosed in (10-4) or the compound A monomethanesulfonate disclosed in (11-4); or the treatment method disclosed in (10-5) or (11-5), where the cancer in which BTK is involved, the cancer in which BTK is overexpressed or activated, or the cancer in which a B cell receptor signal is activated is histiocytic lymphoma, and the cancer in which JAK3 is involved or the cancer in which JAK3 is activation-mutated or activated is acute megakaryocytic leukemia.
   In addition, in still another aspect, the pharmaceutical composition disclosed in (10-1) or (11-1); the use disclosed in (10-2) or (11-2); the use disclosed in (10-3) or (11-3); the compound A disclosed in (10-4) or the compound A monomethanesulfonate disclosed in (11-4); or the treatment method disclosed in (10-5) or (11-5), where the cancer in which BTK is involved, the cancer in which BTK is overexpressed or activated, or the cancer in which a B cell receptor signal is activated is histiocytic lymphoma, and the cancer in which JAK3 is involved or the cancer in which JAK3 is activation-mutated or activated is anaplastic large cell lymphoma.
(13-1) A pharmaceutical composition for treating cancer in which BTK and ITK are involved, comprising the compound A or a pharmaceutically acceptable salt thereof. In one aspect, a pharmaceutical composition for treating cancer in which BTK is overexpressed or activated and cancer in which ITK is activated, comprising the compound A or a pharmaceutically acceptable salt thereof. In another aspect, a pharmaceutical composition for treating cancer in which a B cell receptor signal is activated and cancer in which ITK is activated, comprising the compound A or a pharmaceutically acceptable salt thereof.
(13-2) Use of the compound A or a pharmaceutically acceptable salt thereof for the manufacture of a pharmaceutical composition for treating cancer in which BTK and ITK are involved. In one aspect, use of the compound A or a pharmaceutically acceptable salt thereof for the manufacture of a pharmaceutical composition for treating cancer in which BTK is overexpressed or activated and cancer in which ITK is activated. In another aspect, use of the compound A or a pharmaceutically acceptable salt thereof for the manufacture of a pharmaceutical composition for treating cancer in which a B cell receptor signal is activated and cancer in which ITK is activated.
(13-3) Use of the compound A or a pharmaceutically acceptable salt thereof for treating cancer in which BTK and ITK are involved. In one aspect, use of the compound A or a pharmaceutically acceptable salt thereof for treating cancer in which BTK is overexpressed or activated and cancer in which ITK is activated. In another aspect, use of the compound A or a pharmaceutically acceptable salt thereof for treating cancer in which a B cell receptor signal is activated and cancer in which ITK is activated.
(13-4) The compound A or a pharmaceutically acceptable salt thereof for treating cancer in which BTK and ITK are involved. In one aspect, the compound A or a pharmaceutically acceptable salt thereof for treating cancer in which BTK is overexpressed or activated and cancer in which ITK is activated. In another aspect, the compound A or a pharmaceutically acceptable salt thereof for treating cancer in which a B cell receptor signal is activated and cancer in which ITK is activated.
(13-5) A method for treating cancer in which BTK and ITK are involved, comprising administering an effective amount of the compound A or a pharmaceutically acceptable salt thereof to a subject. In one aspect, a method for treating cancer in which BTK is overexpressed or activated and cancer in which ITK is activated, comprising administering an effective amount of the compound A or a pharmaceutically acceptable salt thereof to a subject. In another aspect, a method for treating cancer in which a B cell receptor signal is activated and cancer in which ITK is activated comprising administering an effective amount of the compound A or a pharmaceutically acceptable salt thereof to a subj ect.
(14-1) A pharmaceutical composition for treating cancer in which BTK and ITK are involved, comprising the compound A monomethanesulfonate. In one aspect, a pharmaceutical composition for treating cancer in which BTK is overexpressed or activated and cancer in which ITK is activated, comprising the compound A monomethanesulfonate. In another aspect, a pharmaceutical composition for treating cancer in which a B cell receptor signal is activated and cancer in which ITK is activated, comprising the compound A monomethanesulfonate.
(14-2) Use of the compound A monomethanesulfonate for the manufacture of a pharmaceutical composition for treating cancer in which BTK and ITK are involved. In one aspect, use of the compound A monomethanesulfonate for the manufacture of a pharmaceutical composition for treating cancer in which BTK is overexpressed or activated and ITK is activated. In another aspect, use of the compound A monomethanesulfonate for the manufacture of a pharmaceutical composition for treating cancer in which a B cell receptor signal is activated and ITK is activated.
(14-3) Use of the compound A monomethanesulfonate for treating cancer in which BTK and ITK are involved. In one aspect, use of the compound A monomethanesulfonate for treating cancer in which BTK is overexpressed or activated and cancer in which ITK is activated. In another aspect, use of the compound A monomethanesulfonate for treating cancer in which a B cell receptor signal is activated and cancer in which ITK is activated.
(14-4) The compound A monomethanesulfonate for treating cancer in which BTK and ITK are involved. In one aspect, the compound A monomethanesulfonate for treating cancer in which BTK is overexpressed or activated and cancer in which ITK is activated. In another aspect, the compound A monomethanesulfonate for treating cancer in which a B cell receptor signal is activated and cancer in which ITK is activated.
(14-5) A method for treating cancer in which BTK and ITK are involved, comprising administering an effective amount of the compound A monomethanesulfonate to a subject. In one aspect, a method for treating cancer in which BTK is overexpressed or activated and cancer in which ITK is activated, comprising administering an effective amount of the compound A monomethanesulfonate to a subject. In another aspect, a method for treating cancer in which a B cell receptor signal is activated and cancer in which ITK is activated comprising administering an effective amount of the compound A monomethanesulfonate to a subject.
(15) The pharmaceutical composition disclosed in (13-1) or (14-1); the use disclosed in (13-2) or (14-2); the use disclosed in (13-3) or (14-3); the compound A disclosed in (13-4) or the compound A monomethanesulfonate disclosed in (14-4); or the treatment method disclosed in (13-5) or (14-5), where the cancer in which BTK is involved, the cancer in which BTK is overexpressed or activated, or the cancer in which a B cell receptor signal is activated is follicular lymphoma, chronic lymphocytic leukemia, small lymphocytic lymphoma, multiple myeloma, mantle cell lymphoma, diffuse large B-cell lymphoma, B-cell prolymphocytic leukemia, acute lymphocytic leukemia, histiocytic lymphoma, or acute myeloid leukemia, and the cancer in which ITK is involved or the cancer in which ITK is activated is acute T cell lymphoma or lymphoblastic leukemia.
   In another aspect, the pharmaceutical composition disclosed in (13-1) or (14-1); the use disclosed in (13-2) or (14-2); the use disclosed in (13-3) or (14-3); the compound A disclosed in (13-4) or the compound A monomethanesulfonate disclosed in (14-4); or the treatment method disclosed in (13-5) or (14-5), where the cancer in which BTK is involved, the cancer in which BTK is overexpressed or activated, or the cancer in which a B cell receptor signal is activated is diffuse large B-cell lymphoma, mantle cell lymphoma, acute myeloid leukemia, or histiocytic lymphoma, and the cancer in which ITK is involved or the cancer in which ITK is activated is acute T cell lymphoma.
   In still another aspect, the pharmaceutical composition disclosed in (13-1) or (14-1); the use disclosed in (13-2) or (14-2); the use disclosed in (13-3) or (14-3); the compound A disclosed in (13-4) or the compound A monomethanesulfonate disclosed in (14-4); or the treatment method disclosed in (13-5) or (14-5), where the cancer in which BTK is involved, the cancer in which BTK is overexpressed or activated, or the cancer in which a B cell receptor signal is activated is diffuse large B-cell lymphoma, mantle cell lymphoma, acute myeloid leukemia, or histiocytic lymphoma, and the cancer in which ITK is involved or the cancer in which ITK is activated is lymphoblastic leukemia.
   In still another aspect, the pharmaceutical composition disclosed in (13-1) or (14-1); the use disclosed in (13-2) or (14-2); the use disclosed in (13-3) or (14-3); the compound A disclosed in (13-4) or the compound A monomethanesulfonate disclosed in (14-4); or the treatment method disclosed in (13-5) or (14-5), where the cancer in which BTK is involved, the cancer in which BTK is overexpressed or activated, or the cancer in which a B cell receptor signal is activated is diffuse large B-cell lymphoma, and the cancer in which ITK is involved or cancer in which ITK is activated is acute T cell lymphoma.
   In still another aspect, the pharmaceutical composition disclosed in (13-1) or (14-1); the use disclosed in (13-2) or (14-2); the use disclosed in (13-3) or (14-3); the compound A disclosed in (13-4) or the compound A monomethanesulfonate disclosed in (14-4); or the treatment method disclosed in (13-5) or (14-5), where the cancer in which BTK is involved, the cancer in which BTK is overexpressed or activated, or the cancer in which a B cell receptor signal is activated is diffuse large B-cell lymphoma, and the cancer in which ITK is involved or the cancer in which ITK is activated is lymphoblastic leukemia.
   In still another aspect, the pharmaceutical composition disclosed in (13-1) or (14-1); the use disclosed in (13-2) or (14-2); the use disclosed in (13-3) or (14-3); the compound A disclosed in (13-4) or the compound A monomethanesulfonate disclosed in (14-4); or the treatment method disclosed in (13-5) or (14-5), where the cancer in which BTK is involved, the cancer in which BTK is overexpressed or activated, or the cancer in which a B cell receptor signal is activated is mantle cell lymphoma, and the cancer in which ITK is involved or the cancer in which ITK is activated is acute T cell lymphoma.
   In still another aspect, the pharmaceutical composition disclosed in (13-1) or (14-1); the use disclosed in (13-2) or (14-2); the use disclosed in (13-3) or (14-3); the compound A disclosed in (13-4) or the compound A monomethanesulfonate disclosed in (14-4); or the treatment method disclosed in (13-5) or (14-5), where the cancer in which BTK is involved, the cancer in which BTK is overexpressed or activated, or the cancer in which a B cell receptor signal is activated is mantle cell lymphoma, and the cancer in which ITK is involved or the cancer in which ITK is activated is lymphoblastic leukemia.
   In still another aspect, the pharmaceutical composition disclosed in (13-1) or (14-1); the use disclosed in (13-2) or (14-2); the use disclosed in (13-3) or (14-3); the compound A disclosed in (13-4) or the compound A monomethanesulfonate disclosed in (14-4); or the treatment method disclosed in (13-5) or (14-5), where the cancer in which BTK is involved, the cancer in which BTK is overexpressed or activated, or the cancer in which a B cell receptor signal is activated is acute myeloid leukemia, and the cancer in which ITK is involved or the cancer in which ITK is activated is acute T cell lymphoma.
   In still another aspect, the pharmaceutical composition disclosed in (13-1) or (14-1); the use disclosed in (13-2) or (14-2); the use disclosed in (13-3) or (14-3); the compound A disclosed in (13-4) or the compound A monomethanesulfonate disclosed in (14-4); or the treatment method disclosed in (13-5) or (14-5), where the cancer in which BTK is involved, the cancer in which BTK is overexpressed or activated, or the cancer in which a B cell receptor signal is activated is acute myeloid leukemia, and the cancer in which ITK is involved or the cancer in which ITK is activated is lymphoblastic leukemia.
   In still another aspect, the pharmaceutical composition disclosed in (13-1) or (14-1); the use disclosed in (13-2) or (14-2); the use disclosed in (13-3) or (14-3); the compound A disclosed in (13-4) or the compound A monomethanesulfonate disclosed in (14-4); or the treatment method disclosed in (13-5) or (14-5), where the cancer in which BTK is involved, the cancer in which BTK is overexpressed or activated, or the cancer in which a B cell receptor signal is activated is histiocytic lymphoma, and the cancer in which ITK is involved or the cancer in which ITK is activated is acute T cell lymphoma.
   In addition, in still another aspect, the pharmaceutical composition disclosed in (13-1) or (14-1); the use disclosed in (13-2) or (14-2); the use disclosed in (13-3) or (14-3); the compound A disclosed in (13-4) or the compound A monomethanesulfonate disclosed in (14-4); or the treatment method disclosed in (13-5) or (14-5), where the cancer in which BTK is involved, the cancer in which BTK is overexpressed or activated, or the cancer in which a B cell receptor signal is activated is histiocytic lymphoma, and the cancer in which ITK is involved or the cancer in which ITK is activated is lymphoblastic leukemia.
(16-1) A pharmaceutical composition for treating cancer in which JAK3 and ITK are involved, comprising the compound A or a pharmaceutically acceptable salt thereof. In one aspect, a pharmaceutical composition for treating cancer in which cancer in which JAK3 is activation-mutated or activated and cancer in which ITK is activated, comprising the compound A or a pharmaceutically acceptable salt thereof.
(16-2) Use of the compound A or a pharmaceutically acceptable salt thereof for the manufacture of a pharmaceutical composition for treating cancer in which JAK3 and ITK are involved. In one aspect, use of the compound A or a pharmaceutically acceptable salt thereof for the manufacture of a pharmaceutical composition for treating cancer in which JAK3 is activation-mutated or activated and cancer in which ITK is activated.
(16-3) Use of the compound A or a pharmaceutically acceptable salt thereof for treating cancer in which JAK3 and ITK are involved. In one aspect, use of the compound A or a pharmaceutically acceptable salt thereof for treating cancer in which JAK3 is activation-mutated or activated and cancer in which ITK is activated.
(16-4) The compound A or a pharmaceutically acceptable salt thereof for treating cancer in which JAK3 and ITK are involved. In one aspect, the compound A or a pharmaceutically acceptable salt thereof for treating cancer in which JAK3 is activation-mutated or activated and cancer in which ITK is activated.
(16-5) A method for treating cancer in which JAK3 and ITK are involved, comprising administering an effective amount of the compound A or a pharmaceutically acceptable salt thereof to a subject. In one aspect, a method for treating cancer in which JAK3 is activation-mutated or activated and cancer in which ITK is activated, comprising administering an effective amount of the compound A or a pharmaceutically acceptable salt thereof to a subject.
(17-1) A pharmaceutical composition for treating cancer in which JAK3 and ITK are involved, comprising the compound A monomethanesulfonate. In one aspect, a pharmaceutical composition for treating cancer in which JAK3 is activation-mutated or activated and cancer in which ITK is activated, comprising the compound A monomethanesulfonate.
(17-2) Use of the compound A monomethanesulfonate for the manufacture of a pharmaceutical composition for treating cancer in which JAK3 and ITK are involved. In one aspect, use of the compound A monomethanesulfonate for the manufacture of a pharmaceutical composition for treating cancer in which JAK3 is activation-mutated or activated and cancer in which ITK is activated.
(17-3) Use of the compound A monomethanesulfonate for treating cancer in which JAK3 and ITK are involved. In one aspect, use of the compound A monomethanesulfonate for treating cancer in which JAK3 is activation-mutated or activated and cancer in which ITK is activated.
(17-4) The compound A monomethanesulfonate for treating cancer in which JAK3 and ITK are involved. In one aspect, the compound A monomethanesulfonate for treating cancer in which JAK3 is activation-mutated or activated and cancer in which ITK is activated.
(17-5) A method for treating cancer in which JAK3 and ITK are involved, comprising administering an effective amount of the compound A monomethanesulfonate to a subject. In one aspect, a method for treating cancer in which JAK3 is activation-mutated or activated and cancer in which ITK is activated, comprising administering an effective amount of the compound A monomethanesulfonate to a subject.
(18) The pharmaceutical composition disclosed in (16-1) or (17-1); the use disclosed in (16-2) or (17-2); the use disclosed in (16-3) or (17-3); the compound A disclosed in (16-4) or the compound A monomethanesulfonate disclosed in (17-4); or the treatment method disclosed in (16-5) or (17-5), where the cancer in which JAK3 is involved or the cancer in which JAK3 is activation-mutated or activated is acute megakaryocytic leukemia or anaplastic large cell lymphoma, and the cancer in which ITK is involved or the cancer in which ITK is activated is acute T cell lymphoma or lymphoblastic leukemia.
   In another aspect, the pharmaceutical composition disclosed in (16-1) or (17-1); the use disclosed in (16-2) or (17-2); the use disclosed in (16-3) or (17-3); the compound A disclosed in (16-4) or the compound A monomethanesulfonate disclosed in (17-4); or the treatment method disclosed in (16-5) or (17-5), where the cancer in which JAK3 is involved or the cancer in which JAK3 is activation-mutated or activated is acute megakaryocytic leukemia, and the cancer in which ITK is involved or the cancer in which ITK is activated is acute T cell lymphoma.
   In still another aspect, the pharmaceutical composition disclosed in (16-1) or (17-1); the use disclosed in (16-2) or (17-2); the use disclosed in (16-3) or (17-3); the compound A disclosed in (16-4) or the compound A monomethanesulfonate disclosed in (17-4); or the treatment method disclosed in (16-5) or (17-5), where the cancer in which JAK3 is involved or the cancer in which JAK3 is activation-mutated or activated is acute megakaryocytic leukemia, and the cancer in which ITK is involved or the cancer in which ITK is activated is lymphoblastic leukemia.
   In still another aspect, the pharmaceutical composition disclosed in (16-1) or (17-1); the use disclosed in (16-2) or (17-2); the use disclosed in (16-3) or (17-3); the compound A disclosed in (16-4) or the compound A monomethanesulfonate disclosed in (17-4); or the treatment method disclosed in (16-5) or (17-5), where the cancer in which JAK3 is involved or the cancer in which JAK3 is activation-mutated or activated is anaplastic large cell lymphoma, and the cancer in which ITK is involved or the cancer in which ITK is activated is acute T cell lymphoma.
   In addition, in still another aspect, the pharmaceutical composition disclosed in (16-1) or (17-1); the use disclosed in (16-2) or (17-2); the use disclosed in (16-3) or (17-3); the compound A disclosed in (16-4) or the compound A monomethanesulfonate disclosed in (17-4); or the treatment method disclosed in (16-5) or (17-5), where the cancer in which JAK3 is involved or the cancer in which JAK3 is activation-mutated or activated is anaplastic large cell lymphoma, and the cancer in which ITK is involved or the cancer in which ITK is activated is lymphoblastic leukemia.
(19-1) A pharmaceutical composition for treating cancer in which BTK, JAK3, and ITK are involved, comprising the compound A or a pharmaceutically acceptable salt thereof. In one aspect, a pharmaceutical composition for treating cancer in which BTK is overexpressed or activated, cancer in which JAK3 is activation-mutated or activated, and cancer in which ITK is activated, comprising the compound A or a pharmaceutically acceptable salt thereof. In another aspect, a pharmaceutical composition for treating cancer in which a B cell receptor signal is activated, cancer in which JAK3 is activation-mutated or activated, and cancer in which ITK is activated, comprising the compound A or a pharmaceutically acceptable salt thereof.
(19-2) Use of the compound A or a pharmaceutically acceptable salt thereof for the manufacture of a pharmaceutical composition for treating cancer in which BTK, JAK3, and ITK are involved. In one aspect, use of the compound A or a pharmaceutically acceptable salt thereof for the manufacture of a pharmaceutical composition for treating cancer in which BTK is overexpressed or activated, cancer in which JAK3 is activation-mutated or activated, and cancer in which ITK is activated. In another aspect, use of the compound A or a pharmaceutically acceptable salt thereof for the manufacture of a pharmaceutical composition for treating cancer in which a B cell receptor signal is activated, cancer in which JAK3 is activation-mutated or activated, and cancer in which ITK is activated.
(19-3) Use of the compound A or a pharmaceutically acceptable salt thereof for treating cancer in which BTK, JAK3, and ITK are involved. In one aspect, use of the compound A or a pharmaceutically acceptable salt thereof for treating cancer in which BTK is overexpressed or activated, cancer in which JAK3 is activation-mutated or activated, and cancer in which ITK is activated. In another aspect, use of the compound A or a pharmaceutically acceptable salt thereof for treating cancer in which a B cell receptor signal is activated, cancer in which JAK3 is activation-mutated or activated, and cancer in which ITK is activated.
(19-4) The compound A or a pharmaceutically acceptable salt thereof for treating cancer in which BTK, JAK3, and ITK are involved. In one aspect, the compound A or a pharmaceutically acceptable salt thereof for treating cancer in which BTK is overexpressed or activated, cancer in which JAK3 is activation-mutated or activated, and cancer in which ITK is activated. In another aspect, the compound A or a pharmaceutically acceptable salt thereof for treating cancer in which a B cell receptor signal is activated, cancer in which JAK3 is activation-mutated or activated, and cancer in which ITK is activated.
(19-5) A method for treating cancer in which BTK, JAK3, and ITK are involved, comprising administering an effective amount of the compound A or a pharmaceutically acceptable salt thereof to a subject. In one aspect, a method for treating cancer in which BTK is overexpressed or activated, cancer in which JAK3 is activation-mutated or activated, and cancer in which ITK is activated, comprising administering an effective amount of the compound A or a pharmaceutically acceptable salt thereof to a subject. In another aspect, a method for treating cancer in which a B cell receptor signal is activated, cancer in which JAK3 is activation-mutated or activated, and cancer in which ITK is activated, comprising administering an effective amount of the compound A or a pharmaceutically acceptable salt thereof to a subject.
(20-1) A pharmaceutical composition for treating cancer in which BTK, JAK3 and ITK are involved, comprising the compound A monomethanesulfonate. In one aspect, a pharmaceutical composition for treating cancer in which BTK is overexpressed or activated, cancer in which JAK3 is activation-mutated or activated, and cancer in which ITK is activated, comprising the compound A monomethanesulfonate. In another aspect, a pharmaceutical composition for treating cancer in which a B cell receptor signal is activated, cancer in which JAK3 is activation-mutated or activated, and cancer in which ITK is activated, comprising the compound A monomethanesulfonate.
(20-2) Use of the compound A monomethanesulfonate for the manufacture of a pharmaceutical composition for treating cancer in which BTK, JAK3, and ITK are involved. In one aspect, use of the compound A monomethanesulfonate for the manufacture of a pharmaceutical composition for treating cancer in which BTK is overexpressed or activated, cancer in which JAK3 is activation-mutated or activated, and cancer in which ITK is activated. In another aspect, use of the compound A monomethanesulfonate for the manufacture of a pharmaceutical composition for treating cancer in which a B cell receptor signal is activated, cancer in which JAK3 is activation-mutated or activated, and cancer in which ITK is activated.
(20-3) Use of the compound A monomethanesulfonate for treating cancer in which BTK, JAK3, and ITK are involved. In one aspect, use of the compound A monomethanesulfonate for treating cancer in which BTK is overexpressed or activated, cancer in which JAK3 is activation-mutated or activated, and cancer in which ITK is activated. In another aspect, use of the compound A monomethanesulfonate for treating cancer in which a B cell receptor signal is activated, cancer in which JAK3 is activation-mutated or activated, and cancer in which ITK is activated.
(20-4) The compound A monomethanesulfonate for treating cancer in which BTK, JAK3, and ITK are involved. In one aspect, the compound A monomethanesulfonate for treating cancer in which BTK is overexpressed or activated, cancer in which JAK3 is activation-mutated or activated, and cancer in which ITK is activated. In another aspect, the compound A monomethanesulfonate for treating cancer in which a B cell receptor signal is activated, cancer in which JAK3 is activation-mutated or activated, and cancer in which ITK is activated.
(20-5) A method for treating cancer in which BTK, JAK3, and ITK are involved, comprising administering an effective amount of the compound A monomethanesulfonate to a subject. In one aspect, a method for treating cancer in which BTK is overexpressed or activated, cancer in which JAK3 is activation-mutated or activated, and cancer in which ITK is activated, comprising administering an effective amount of the compound A monomethanesulfonate to a subject. In another aspect, a method for treating cancer in which a B cell receptor signal is activated, cancer in which JAK3 is activation-mutated or activated, and cancer in which ITK is activated, comprising administering an effective amount of the compound A monomethanesulfonate to a subject.
(21) The pharmaceutical composition disclosed in (19-1) or (20-1); the use disclosed in (19-2) or (20-2); the use disclosed in (19-3) or (20-3); the compound A disclosed in (19-4) or the compound A monomethanesulfonate disclosed in (20-4); or the treatment method disclosed in (19-5) or (20-5), where the cancer in which BTK is involved, the cancer in which BTK is overexpressed or activated, or the cancer in which a B cell receptor signal is activated is follicular lymphoma, chronic lymphocytic leukemia, small lymphocytic lymphoma, multiple myeloma, mantle cell lymphoma, diffuse large B-cell lymphoma, B-cell prolymphocytic leukemia, acute lymphocytic leukemia, histiocytic lymphoma, or acute myeloid leukemia, the cancer in which JAK3 is involved or the cancer in which JAK3 is activation-mutated or activated is acute megakaryocytic leukemia or anaplastic large cell lymphoma, and the cancer in which ITK is involved or the cancer in which ITK is activated is acute T cell lymphoma or lymphoblastic leukemia.

In another aspect, the pharmaceutical composition disclosed in (19-1) or (20-1); the use disclosed in (19-2) or (20-2); the use disclosed in (19-3) or (20-3); the compound A disclosed in (19-4) or the compound A monomethanesulfonate disclosed in (20-4); or the treatment method disclosed in (19-5) or (20-5), where the cancer in which BTK is involved, the cancer in which BTK is overexpressed or activated, or the cancer in which a B cell receptor signal is activated is diffuse large B-cell lymphoma, mantle cell lymphoma, acute myeloid leukemia, or histiocytic lymphoma, the cancer in which JAK3 is involved or the cancer in which JAK3 is activation-mutated or activated is acute megakaryocytic leukemia or anaplastic large cell lymphoma, and the cancer in which ITK is involved or the cancer in which ITK is activated is acute T cell lymphoma or lymphoblastic leukemia.

In still another aspect, the pharmaceutical composition disclosed in (19-1) or (20-1); the use disclosed in (19-2) or (20-2); the use disclosed in (19-3) or (20-3); the compound A disclosed in (19-4) or the compound A monomethanesulfonate disclosed in (20-4); or the treatment method disclosed in (19-5) or (20-5), where the cancer in which BTK is involved, the cancer in which BTK is overexpressed or activated, or the cancer in which a B cell receptor signal is activated is diffuse large B-cell lymphoma, mantle cell lymphoma, acute myeloid leukemia, or histiocytic lymphoma, the cancer in which JAK3 is involved or the cancer in which JAK3 is activation-mutated or activated is acute megakaryocytic leukemia, and the cancer in which ITK is involved or the cancer in which ITK is activated is acute T cell lymphoma.

In still another aspect, the pharmaceutical composition disclosed in (19-1) or (20-1); the use disclosed in (19-2) or (20-2); the use disclosed in (19-3) or (20-3); the compound A disclosed in (19-4) or the compound A monomethanesulfonate disclosed in (20-4); or the treatment method disclosed in (19-5) or (20-5), where the cancer in which BTK is involved, the cancer in which BTK is overexpressed or activated, or the cancer in which a B cell receptor signal is activated is diffuse large B-cell lymphoma, mantle cell lymphoma, acute myeloid leukemia, or histiocytic lymphoma, the cancer in which JAK3 is involved or the cancer in which JAK3 is activation-mutated or activated is acute megakaryocytic leukemia, and the cancer in which ITK is involved or the cancer in which ITK is activated is lymphoblastic leukemia.

In still another aspect, the pharmaceutical composition disclosed in (19-1) or (20-1); the use disclosed in (19-2) or (20-2); the use disclosed in (19-3) or (20-3); the compound A disclosed in (19-4) or the compound A monomethanesulfonate disclosed in (20-4); or the treatment method disclosed in (19-5) or (20-5), where the cancer in which BTK is involved, the cancer in which BTK is overexpressed or activated, or the cancer in which a B cell receptor signal is activated is diffuse large B-cell lymphoma, mantle cell lymphoma, acute myeloid leukemia, or histiocytic lymphoma, the cancer in which JAK3 is involved or the cancer in which JAK3 is activation-mutated or activated is anaplastic large cell lymphoma, and the cancer in which ITK is involved or the cancer in which ITK is activated is acute T cell lymphoma.

In still another aspect, the pharmaceutical composition disclosed in (19-1) or (20-1); the use disclosed in (19-2) or (20-2); the use disclosed in (19-3) or (20-3); the compound A disclosed in (19-4) or the compound A monomethanesulfonate disclosed in (20-4); or the treatment method disclosed in (19-5) or (20-5), where the cancer in which BTK is involved, the cancer in which BTK is overexpressed or activated, or the cancer in which a B cell receptor signal is activated is diffuse large B-cell lymphoma, mantle cell lymphoma, acute myeloid leukemia, or histiocytic lymphoma, the cancer in which JAK3 is involved or the cancer in which JAK3 is activation-mutated or activated is anaplastic large cell lymphoma, and the cancer in which ITK is involved or the cancer in which ITK is activated is lymphoblastic leukemia.

In still another aspect, the pharmaceutical composition disclosed in (19-1) or (20-1); the use disclosed in (19-2) or (20-2); the use disclosed in (19-3) or (20-3); the compound A disclosed in (19-4) or the compound A monomethanesulfonate disclosed in (20-4); or the treatment method disclosed in (19-5) or (20-5), where the cancer in which BTK is involved, the cancer in which BTK is overexpressed or activated, or the cancer in which a B cell receptor signal is activated is diffuse large B-cell lymphoma, the cancer in which JAK3 is involved or the cancer in which JAK3 is activation-mutated or activated is acute megakaryocytic leukemia, and the cancer in which ITK is involved or the cancer in which ITK is activated is acute T cell lymphoma.

In still another aspect, the pharmaceutical composition disclosed in (19-1) or (20-1); the use disclosed in (19-2) or (20-2); the use disclosed in (19-3) or (20-3); the compound A disclosed in (19-4) or the compound A monomethanesulfonate disclosed in (20-4); or the treatment method disclosed in (19-5) or (20-5), where the cancer in which BTK is involved, the cancer in which BTK is overexpressed or activated, or the cancer in which a B cell receptor signal is activated is diffuse large B-cell lymphoma, the cancer in which JAK3 is involved or the cancer in which JAK3 is activation-mutated or activated is acute megakaryocytic leukemia, and the cancer in which ITK is involved or the cancer in which ITK is activated is lymphoblastic leukemia.

In still another aspect, the pharmaceutical composition disclosed in (19-1) or (20-1); the use disclosed in (19-2) or (20-2); the use disclosed in (19-3) or (20-3); the compound A disclosed in (19-4) or the compound A monomethanesulfonate disclosed in (20-4); or the treatment method disclosed in (19-5) or (20-5), where the cancer in which BTK is involved, the cancer in which BTK is overexpressed or activated, or the cancer in which a B cell receptor signal is activated is diffuse large B-cell lymphoma, the cancer in which JAK3 is involved or the cancer in which JAK3 is activation-mutated or activated is anaplastic large cell lymphoma, and the cancer in which ITK is involved or the cancer in which ITK is activated is acute T cell lymphoma.

In still another aspect, the pharmaceutical composition disclosed in (19-1) or (20-1); the use disclosed in (19-2) or (20-2); the use disclosed in (19-3) or (20-3); the compound A disclosed in (19-4) or the compound A monomethanesulfonate disclosed in (20-4); or the treatment method disclosed in (19-5) or (20-5), where the cancer in which BTK is involved, the cancer in which BTK is overexpressed or activated, or the cancer in which a B cell receptor signal is activated is diffuse large B-cell lymphoma, the cancer in which JAK3 is involved or the cancer in which JAK3 is activation-mutated or activated is anaplastic large cell lymphoma, and the cancer in which ITK is involved or the cancer in which ITK is activated is lymphoblastic leukemia.

In still another aspect, the pharmaceutical composition disclosed in (19-1) or (20-1); the use disclosed in (19-2) or (20-2); the use disclosed in (19-3) or (20-3); the compound A disclosed in (19-4) or the compound A monomethanesulfonate disclosed in (20-4); or the treatment method disclosed in (19-5) or (20-5), where the cancer in which BTK is involved, the cancer in which BTK is overexpressed or activated, or the cancer in which a B cell receptor signal is activated is mantle cell lymphoma, the cancer in which JAK3 is involved or the cancer in which JAK3 is activation-mutated or activated is acute megakaryocytic leukemia, and the cancer in which ITK is involved or the cancer in which ITK is activated is acute T cell lymphoma.

In still another aspect, the pharmaceutical composition disclosed in (19-1) or (20-1); the use disclosed in (19-2) or (20-2); the use disclosed in (19-3) or (20-3); the compound A disclosed in (19-4) or the compound A monomethanesulfonate disclosed in (20-4); or the treatment method disclosed in (19-5) or (20-5), where the cancer in which BTK is involved, the cancer in which BTK is overexpressed or activated, or the cancer in which a B cell receptor signal is activated is mantle cell lymphoma, the cancer in which JAK3 is involved or the cancer in which JAK3 is activation-mutated or activated is acute megakaryocytic leukemia, and the cancer in which ITK is involved or the cancer in which ITK is activated is lymphoblastic leukemia.

In still another aspect, the pharmaceutical composition disclosed in (19-1) or (20-1); the use disclosed in (19-2) or (20-2); the use disclosed in (19-3) or (20-3); the compound A disclosed in (19-4) or the compound A monomethanesulfonate disclosed in (20-4); or the treatment method disclosed in (19-5) or (20-5), where the cancer in which BTK is involved, the cancer in which BTK is overexpressed or activated, or the cancer in which a B cell receptor signal is activated is mantle cell lymphoma, the cancer in which JAK3 is involved or the cancer in which JAK3 is activation-mutated or activated is anaplastic large cell lymphoma, and the cancer in which ITK is involved or the cancer in which ITK is activated is acute T cell lymphoma.

In still another aspect, the pharmaceutical composition disclosed in (19-1) or (20-1); the use disclosed in (19-2) or (20-2); the use disclosed in (19-3) or (20-3); the compound A disclosed in (19-4) or the compound A monomethanesulfonate disclosed in (20-4); or the treatment method disclosed in (19-5) or (20-5), where the cancer in which BTK is involved, the cancer in which BTK is overexpressed or activated, or the cancer in which a B cell receptor signal is activated is mantle cell lymphoma, the cancer in which JAK3 is involved or the cancer in which JAK3 is activation-mutated or activated is anaplastic large cell lymphoma, and the cancer in which ITK is involved or the cancer in which ITK is activated is lymphoblastic leukemia.

In still another aspect, the pharmaceutical composition disclosed in (19-1) or (20-1); the use disclosed in (19-2) or (20-2); the use disclosed in (19-3) or (20-3); the compound A disclosed in (19-4) or the compound A monomethanesulfonate disclosed in (20-4); or the treatment method disclosed in (19-5) or (20-5), where the cancer in which BTK is involved, the cancer in which BTK is overexpressed or activated, or the cancer in which a B cell receptor signal is activated is acute myeloid leukemia, the cancer in which JAK3 is involved or the cancer in which JAK3 is activation-mutated or activated is acute megakaryocytic leukemia, and the cancer in which ITK is involved or the cancer in which ITK is activated is acute T cell lymphoma.

In still another aspect, the pharmaceutical composition disclosed in (19-1) or (20-1); the use disclosed in (19-2) or (20-2); the use disclosed in (19-3) or (20-3); the compound A disclosed in (19-4) or the compound A monomethanesulfonate disclosed in (20-4); or the treatment method disclosed in (19-5) or (20-5), where the cancer in which BTK is involved, the cancer in which BTK is overexpressed or activated, or the cancer in which a B cell receptor signal is activated is acute myeloid leukemia, the cancer in which JAK3 is involved or the cancer in which JAK3 is activation-mutated or activated is acute megakaryocytic leukemia, and the cancer in which ITK is involved or the cancer in which ITK is activated is lymphoblastic leukemia.

In still another aspect, the pharmaceutical composition disclosed in (19-1) or (20-1); the use disclosed in (19-2) or (20-2); the use disclosed in (19-3) or (20-3); the compound A disclosed in (19-4) or the compound A monomethanesulfonate disclosed in (20-4); or the treatment method disclosed in (19-5) or (20-5), where the cancer in which BTK is involved, the cancer in which BTK is overexpressed or activated, or the cancer in which a B cell receptor signal is activated is acute myeloid leukemia, the cancer in which JAK3 is involved or the cancer in which JAK3 is activation-mutated or activated is anaplastic large cell lymphoma, and the cancer in which ITK is involved or the cancer in which ITK is activated is acute T cell lymphoma.

In still another aspect, the pharmaceutical composition disclosed in (19-1) or (20-1); the use disclosed in (19-2) or (20-2); the use disclosed in (19-3) or (20-3); the compound A disclosed in (19-4) or the compound A monomethanesulfonate disclosed in (20-4); or the treatment method disclosed in (19-5) or (20-5), where the cancer in which BTK is involved, the cancer in which BTK is overexpressed or activated, or the cancer in which a B cell receptor signal is activated is acute myeloid leukemia, the cancer in which JAK3 is involved or the cancer in which JAK3 is activation-mutated or activated is anaplastic large cell lymphoma, and the cancer in which ITK is involved or the cancer in which ITK is activated is lymphoblastic leukemia.

In still another aspect, the pharmaceutical composition disclosed in (19-1) or (20-1); the use disclosed in (19-2) or (20-2); the use disclosed in (19-3) or (20-3); the compound A disclosed in (19-4) or the compound A monomethanesulfonate disclosed in (20-4); or the treatment method disclosed in (19-5) or (20-5), where the cancer in which BTK is involved, the cancer in which BTK is overexpressed or activated, or the cancer in which a B cell receptor signal is activated is histiocytic lymphoma, the cancer in which JAK3 is involved or the cancer in which JAK3 is activation-mutated or activated is acute megakaryocytic leukemia, and the cancer in which ITK is involved or the cancer in which ITK is activated is acute T cell lymphoma.

In still another aspect, the pharmaceutical composition disclosed in (19-1) or (20-1); the use disclosed in (19-2) or (20-2); the use disclosed in (19-3) or (20-3); the compound A disclosed in (19-4) or the compound A monomethanesulfonate disclosed in (20-4); or the treatment method disclosed in (19-5) or (20-5), where the cancer in which BTK is involved, the cancer in which BTK is overexpressed or activated, or the cancer in which a B cell receptor signal is activated is histiocytic lymphoma, the cancer in which JAK3 is involved or the cancer in which JAK3 is activation-mutated or activated is acute megakaryocytic leukemia, and the cancer in which ITK is involved or the cancer in which ITK is activated is lymphoblastic leukemia.

In still another aspect, the pharmaceutical composition disclosed in (19-1) or (20-1); the use disclosed in (19-2) or (20-2); the use disclosed in (19-3) or (20-3); the compound A disclosed in (19-4) or the compound A monomethanesulfonate disclosed in (20-4); or the treatment method disclosed in (19-5) or (20-5), where the cancer in which BTK is involved, the cancer in which BTK is overexpressed or activated, or the cancer in which a B cell receptor signal is activated is histiocytic lymphoma, the cancer in which JAK3 is involved or the cancer in which JAK3 is activation-mutated or activated is anaplastic large cell lymphoma, and the cancer in which ITK is involved or the cancer in which ITK is activated is acute T cell lymphoma.

In still another aspect, the pharmaceutical composition disclosed in (19-1) or (20-1); the use disclosed in (19-2) or (20-2); the use disclosed in (19-3) or (20-3); the compound A disclosed in (19-4) or the compound A monomethanesulfonate disclosed in (20-4); or the treatment method disclosed in (19-5) or (20-5), where the cancer in which BTK is involved, the cancer in which BTK is overexpressed or activated, or the cancer in which a B cell receptor signal is activated is histiocytic lymphoma, the cancer in which JAK3 is involved or the cancer in which JAK3 is activation-mutated or activated is anaplastic large cell lymphoma, and the cancer in which ITK is involved or the cancer in which ITK is activated is lymphoblastic leukemia.

The compound A or a pharmaceutically acceptable salt thereof can be available according to the method disclosed in the aforementioned Patent Document 1 (WO 2013/108754) or a modified method thereof.

In addition, the "pharmaceutically acceptable salt of the compound A" means an acid addition salt of the compound A and examples thereof include an acid addition salt of inorganic acids such as hydrochloric acid, hydrobromic acid, hydroiodic acid, sulfuric acid, nitric acid, and phosphoric acid, and organic acids such as formic acid, acetic acid, propionic acid, oxalic acid, malonic acid, succinic acid, fumaric acid, maleic acid, lactic acid, malic acid, mandelic acid, tartaric acid, dibenzoyl tartaric acid, ditoluoyl tartaric acid, citric acid, methane sulfonic acid, ethane sulfonic acid, benzene sulfonic acid, p-toluene sulfonic acid, aspartic acid, and glutamine acid. In addition, the "pharmaceutically acceptable salt of the compound A" includes a solvate of the compound A, specifically, for example, includes a hydrate or ethanolate, and further includes an acid addition salt of the compound A.

In addition, examples of the "compound A or a pharmaceutically acceptable salt thereof" in one aspect include the compound A (free base) and the compound A monomethanesulfonate in another aspect.

The pharmaceutical composition containing the compound A or a pharmaceutically acceptable salt thereof can be prepared using an excipient which is commonly used in the art, that is, an excipient for pharmaceutical preparation, a carrier for pharmaceutical preparation, or the like, in accordance with the methods which are commonly used in the art.

The administration of the pharmaceutical composition may be oral administration via tablets, pills, capsules, granules, powders and liquids, or parenteral administration via injections such as intraarticular, intravenous, and intramuscular injections, suppositories, transdermal liquids, ointments, transdermal patches, transmucosal liquids, transmucosal patches, inhalers, or the like.

As a solid composition for oral administration, tablets, powders, and granules are used. In this solid composition, one or more active ingredients are mixed with at least one inactive excipient. In a conventional method, the composition may contain inactive additives, for example, lubricants, disintegrating agents, stabilizers, and solubilization assisting agents. The tablets or pills may be coated with sugar or gastric- or enteric-soluble substance films.

A liquid composition for oral administration includes pharmaceutically acceptable emulsions, solutions, suspensions, syrups, elixirs, or the like. The liquid composition contains generally used inert diluents, for example, purified water or ethanol, and further contains auxiliary agents such as solubilization assisting agents, moistening agents, and suspending agents, sweeteners, flavors, aromatics, or antiseptics.

Injections for parenteral administration include sterile aqueous or non-aqueous solutions, suspensions, or emulsions. Aqueous solvents include, for example, distilled water for injection or physiological saline. Examples of non-aqueous solvents include alcohols such as ethanol. Such compositions may further contain tonicity agents, antiseptics, moistening agents, emulsifying agents, dispersing agents, stabilizers, or solubilization assisting agents. These are sterilized, for example, by filtration through bacteria retaining filter, blending of bactericide, or irradiation. In addition, these can also be used by preparing sterile solid compositions, and dissolving or suspending in sterile water or sterile solvents for injection prior to use.

In general, for oral administration, the daily dose is about 0.001 mg/kg to 100 mg/kg, preferably 0.01 mg/kg to 30 mg/kg, and more preferably 0.1 to 10 mg/kg, per body weight, administered in one portion or in 2 to 4 separate portions. In the case of intravenous administration, the daily dose is suitably administered from about 0.0001 mg/kg to 10 mg/kg per body weight, once a day or two or more times a day. In addition, a transmucosal agent is administered at a dose from about 0.001 mg/kg to 100 mg/kg per body weight, once a day or two or more times a day. Doses are determined as appropriate depending on the individual in consideration of the symptoms, age, gender, and the like.

Although varying depending on administration routes, formulations, administration sites, or the types of excipients or additives, the pharmaceutical composition of the present invention contains 0.01% by weight to 99% by weight, and in one aspect, 0.01% by weight to 50% by weight of the compound A or a pharmaceutically acceptable salt thereof as an active ingredient.

The pharmaceutical composition of the present invention can be used in combination with various therapeutic agents that are considered effective for cancer, in particular, cancer in which one or more kinases of JAK3 and ITK are involved. The combined preparations may be administered simultaneously, or separately and continuously, or at a desired time interval. The preparations to be administered simultaneously may be a mixture, or may be prepared individually. In particular, examples of the agents that can be used in combination herein include an anti CD20 monoclonal antibody such as rituximab, an alkylating agent such as cyclophosphamide and ifosfamide, an antineoplastic drug such as doxorubicin, cisplatin, methotrexate, anthracycline, and lenalidomide, a microtube polymerization inhibitor such as vincristine, corticosteroid such as prednisone, prednisolone, and dexamethasone, an antimetabolite such as bendamustine, a kinase inhibitor such as imatinib and dasatinib, a cytokine formulation such as interferon, antibiotics such as mitoxantrone and bleomycin, and asparaginase.

### Examples

The pharmacological effects of the pharmaceutical composition of the present invention were confirmed by Examples below. In addition, a compound A monomethanesulfonate (hereinafter, referred to as "test compound B") was used as the compound A or a pharmaceutically acceptable salt thereof in the following Examples. In each Example, the concentration of the test compound B was calculated in terms of the concentration of the compound A free form.

### Example 1 Evaluation of BTK Kinase Inhibitory Activity

The BTK kinase inhibitory activity was evaluated using QSS Assist BTK_MSA kit (Carna Bioscience).

The test compound B was dissolved in dimethyl sulfoxide (DMSO) to prepare a solution at a 100-fold concentration as compared to the final concentration. The solution was also 25-fold diluted with an appurtenant assay buffer to obtain a test compound B solution. In addition, BTK kinase appurtenant to the kit was used for the reaction.

5 µL of the test compound B solution at a 4-fold concentration, prepared using the aforementioned assay buffer, and 10 µL of a BTK kinase solution at a 2-fold concentration were mixed within a well of a 384 well plate, and left to undergo a reaction at room temperature for 30 minutes. Continuously, 5 µL of a substrate peptide/ATP/MgCl₂ solution at a 4-fold concentration was added thereto to perform the reaction at room temperature for 1 hour. The substrate peptide was used at a final concentration of 1000 nM, ATP at a final concentration of 1 mM, and MgCl₂ at a final concentration of 5 mM. Thereafter, 60 µlL of an appurtenant termination buffer was added thereto to stop the reaction. The BTK kinase activity was calculated by quantifying a product conversion ratio obtained from the substrate peptide peak height and the product (phosphorylated substrate peptide) peak height with LabChip EZ Reader II (PerkinElmer).

For the data analysis, by defining the average conversion ratio of a control well including all the reaction components as 0% inhibition and defining the average conversion ratio of a background well including all the reaction components except for the BTK kinase as 100% inhibition, the inhibition rate was calculated from the average conversion ratio of the wells for testing the respective test compound B. The IC₅₀ value was determined by nonlinear regression based on the test compound B concentrations and the plots from the inhibition rates.

As a result, the test compound B inhibited the BTK kinase activity with an IC₅₀ value of 0.17 nM.

### Example 2 Evaluation of Proliferation Inhibition using Human Diffuse Large B-cell Lymphoma Cell Line OCI-Ly10 Cells

OCI-Ly10 is a cell line originated from human diffuse large B-cell lymphoma, which is a B-cell malignant neoplasm, and the BTK dependency has been confirmed (Nature. 2010; 463:88-92). The human diffuse large B-cell lymphoma cell line OCI-Ly10 cells (University Health Network) cultured in an Iscove's Modified Dulbecco's Medium (Sigma) containing a 20% bovine serum were seeded into 96 well plates. On the same day, DMSO solutions of the test compound B at a final concentrations from 0.1 nM to 1 µM or only DMSO (DMSO group) were added thereto to culture the cells at 37 °C in 5% CO₂ for 3 days. Thereafter, the cell number was measured using a cell counting reagent (CellTiter-Glo (registered trademark) Luminescent Cell Viability Assay (Promega)). The cell proliferation inhibition rate was calculated by the measured value in the DMSO group set as 0% inhibition and the measured value in the DMSO group on day of cell seeding set as 100% inhibition.

As a result, the test compound B inhibited the proliferation of the OCI-Ly10 cells with a GI₅₀ value of 1.0 nM.

### Example 3 Evaluation of Proliferation Inhibition using Human Mantle Cell Lymphoma Cell Line Rec-1 Cells

Rec-1 is a cell line originated from human mantle cell lymphoma, a kind of B-cell malignant neoplasm, and the BTK expression and phosphorylation have been confirmed (Cell Oncol. 2011; 34:141-153). The human mantle cell lymphoma cell line Rec-1 cells (American Type Culture Collection, CRL-3004) cultured in RPMI1640 medium (Sigma) containing 10% bovine serum were seeded into 96 well plates. On the next day, DMSO solutions of the test compound B at final concentrations from 1 nM to 10 µM or only DMSO (DMSO group) were added thereto to culture the cells at 37 °C in 5% CO₂ for 3 days. Thereafter, the cell number was measured using a cell counting reagent (CellTiter-Glo (registered trademark) Luminescent Cell Viability Assay (Promega)). The cell proliferation inhibition rate was calculated as the measured value in the DMSO group set as 0% inhibition and the measured value in the well only with the medium on which the cells have not been seeded set as 100% inhibition.

As a result, the test compound B inhibited the proliferation of the Rec-1 cells with an IC₅₀ value of 68 nM.

### Example 4 Evaluation of Proliferation Inhibition using Human Acute Myeloid Leukemia Cell Line KG-1 Cells

KG-1 is a cell line originated from human acute myeloid leukemia and the BTK expression has been confirmed (J. Immunol. 1994; 152(2):557-565). The human acute myeloid leukemia cell line KG-1cells (American Type Culture Collection, CCL-246) cultured in RPMI1640 medium (Sigma) containing 10% bovine serum were seeded into 96 well plates. On the next day, DMSO solutions of the test compound B at final concentrations from 1 nM to 10 µM or only DMSO (DMSO group) were added thereto to culture the cells at 37 °C in 5% CO₂ for 3 days. Thereafter, the cell number was measured using a cell counting reagent (CellTiter-Glo (registered trademark) Luminescent Cell Viability Assay (Promega)). The cell proliferation inhibition rate was calculated as the measured value in the DMSO group set as 0% inhibition and the measured value in the well only with the medium on which the cells have not been seeded set as 100% inhibition.

As a result, the test compound B inhibited the proliferation of the KG-1 cells with an IC₅₀ value of 38 nM.

### Example 5 Evaluation of Proliferation Inhibition using Human Acute Myeloid Leukemia Cell Line MV4-11 Cells

MV4-11 is a cell line originated from human acute myeloid leukemia and the BTK expression has been confirmed (Mol. Cell Proteomics. 2009; 8(7):1751-1764). The human acute myeloid leukemia cell line MV4-11 cells (American Type Culture Collection, CRL-9591) cultured in an Iscove's Modified Dulbecco's Medium (Sigma) contain myeloid ing 10% bovine serum were seeded into 96 well plates. On the next day, DMSO solution of the test compound B at final concentrations from 1 nM to 10 µM or only DMSO (DMSO group) were added thereto to culture the cells at 37 °C in 5% CO₂ for 3 days. Thereafter, the cell number was measured using a cell counting reagent (CellTiter-Glo (registered trademark) Luminescent Cell Viability Assay (Promega)). The cell proliferation inhibition rate was calculated as the measured value in the DMSO group set as 0% inhibition and the measured value in the well only with the medium on which the cells have not been seeded set as 100% inhibition.

As a result, the test compound B inhibited the proliferation of the MV4-11 cells with an IC₅₀ value of 10 nM.

### Example 6 Evaluation of Proliferation Inhibition using Human Histiocytic Lymphoma Cell Line U-937 Cells

U-937 is a cell line originated from human histiocytic lymphoma and the BTK expression has been confirmed (J. Immunol. 1994; 152(2):557-565). The human histiocytic lymphoma cell line U-937 cells (Sumitomo Dainippon Pharma Co., Ltd.) cultured in RPMI1640 medium (Sigma) containing 10% bovine serum were seeded into 96 well plates. On the same day, DMSO solutions of the test compound B at a final concentrations from 1 nM to 10 µM or only DMSO (DMSO group) were added thereto to culture the cells at 37 °C in 5% CO₂ for 3 days. Thereafter, the cell number was measured using a cell counting reagent (CellTiter-Glo (registered trademark) Luminescent Cell Viability Assay (Promega)). The cell proliferation inhibition rate was calculated as the measured value in the DMSO group set as 0% inhibition and the measured value in the well only with the medium on which the cells have not been seeded set as 100% inhibition.

As a result, the test compound B inhibited the proliferation of the U-937 cells with an IC₅₀ value of 63 nM.

### Example 7 Anti-Tumor Evaluation in OCI-Ly10 Subcutaneous Xenograft Mouse Model

The human diffuse large B-cell lymphoma cell line OCI-Ly10 cells were subcutaneously implanted to a dorsal part of an immunodeficient mouse (NOD.CB 17-Prkdc scid/J, male, 4-week-old (Charles River Laboratories Japan, Inc.)) to prepare an OCI-Ly10 subcutaneous xenograft mouse model. To mice which were divided based on the tumor volumes, 0.5% methylcellulose (control group: 10 mL/kg/day, QD, po (n=4)) or the test compound B (test compound B-administered group: 30 or 100 mg/kg/day in 0.5% methylcellulose suspension, QD, po (n=4)) were administered. The change in the tumor volume was evaluated over time.

As a result, in the control group, the tumor volume having a size of 188 mm³ on the day when the administration was started was increased to a size of 520 mm³ after 14 days. Meanwhile, in the test compound B at 30 mg/kg/day administered group, the tumor volume having a size of 190 mm³ on the day when the administration was started was reduced to a size of 50 mm³ after 14 days. Further, in the test compound B at 100 mg/kg/day administered group, the size of the tumor volume was reduced from 184 mm³ to 22 mm³.

### Example 8 Evaluation of JAK3 Kinase Inhibitory Activity

The JAK3 kinase inhibitory activity was evaluated using QSS Assist JAK3_MSA kit (Carna Bioscience).

The test compound B was dissolved in dimethyl sulfoxide (DMSO) to prepare a solution at a 100-fold concentration as compared to the test concentration. The solution was also 25-fold diluted with an appurtenant assay buffer to obtain a test compound B solution. In addition, JAK3 kinase appurtenant to the kit was used for the reaction.

5 µL of the test compound B solution at a 4-fold concentration, prepared using the aforementioned assay buffer, and 10 µL of a JAK3 kinase solution at a 2-fold concentration were mixed within a well of a 384 well plate, and incubated at room temperature for 30 minutes. Continuously, 5 µL of a substrate peptide/ATP/MgCl₂ solution at a 4-fold concentration was added thereto to perform the reaction at room temperature for 1 hour. The substrate peptide was used at a final concentration of 1 µM, ATP at a final concentration of 1 mM, and MgCl₂ at a final concentration of 5 mM. Thereafter, 60 µL of an appurtenant termination buffer was added thereto to stop the reaction. The JAK3 kinase activity was calculated by quantifying a product conversion ratio obtained from the substrate peptide peak height and the product (phosphorylated substrate peptide) peak height with LabChip EZ Reader II (PerkinElmer).

For the data analysis, by defining the average conversion ratio of a control well including all the reaction components as 0% inhibition and defining the average conversion ratio of a background well including all the reaction components except for the JAK3 kinase as 100% inhibition, the inhibition rate was calculated from the average conversion ratio of the wells for testing the respective test compound B. The IC₅₀ value was determined by nonlinear regression based on the test compound B concentrations and the plots from the inhibition rates.

As a result, the test compound B inhibited the JAK3 kinase activity with an IC₅₀ value of 0.44 nM.

### Example 9 Evaluation of Proliferation Inhibition using Human Anaplastic Large Cell Lymphoma Cell Line SU-DHL-1 Cells

SU-DHL-1 is a cell line originated from human anaplastic large cell lymphoma and the activation of JAK 3 has been confirmed (Leukemia. 2014; 28:941-944). The human anaplastic large cell lymphoma cell line SU-DHL-1 cells (American Type Culture Collection, CRL-2955) cultured in RPMI1640 medium (Sigma) containing 10% bovine serum were seeded into 96 well plates. On the same day, DMSO solutions of the test compound B at a final concentrations from 1 nM to 10 µM or only DMSO (DMSO group) was added thereto to culture the cells at 37 °C in 5% CO₂ for 4 days. Thereafter, the cell number was measured using a cell counting reagent (CellTiter-Glo (registered trademark) Luminescent Cell Viability Assay (Promega)). The cell proliferation inhibition rate was calculated as the measured value in the DMSO group set as 0% inhibition and the measured value in the well only with the medium on which the cells have not been seeded set as 100% inhibition.

As a result, the test compound B inhibited the proliferation of the SU-DHL-1 cells with an IC₅₀ value of 120 nM.

### Example 10 Evaluation of Proliferation Inhibition using Human Anaplastic Large Cell Lymphoma Cell Line KARPAS-299 Cells

KARPAS-299 is a cell line originated from human anaplastic large cell lymphoma and the activation of JAK 3 has been confirmed (Oncogene. 2002; 21:1038-1047). The human anaplastic large cell lymphoma cell line KARPAS-299 cells (DSMZ, ACC31) cultured in RPMI1640 medium (Sigma) containing a 10% bovine serum were seeded into 96 well plates. On the same day, DMSO solution of the test compound B at a final concentrations from 1 nM to 10 µM or only DMSO (DMSO group) were added thereto to culture the cells at 37 °C in 5% CO₂ for 3 days. Thereafter, the cell number was measured using a cell counting reagent (CellTiter-Glo (registered trademark) Luminescent Cell Viability Assay (Promega)). The cell proliferation inhibition rate was calculated as the measured value in the DMSO group set as 0% inhibition and the measured value in the well only with the medium on which the cells have not been seeded set as 100% inhibition.

As a result, the test compound B inhibited the proliferation of the KARPAS-299 cells and the IC₅₀ value was 250 nM.

### Example 11 Evaluation of Proliferation Inhibition using Human Acute Megakaryocytic Leukemia Cell Line CMK Cells

CMK is a cell line originated from human acute megakaryocytic leukemia and the activation of JAK 3 has been confirmed (Cancer Cell. 2006; 10:65-75). The human acute megakaryocytic leukemia cell line CMK cells (DSMZ, ACC392) cultured in RPMI1640 medium (Sigma) containing 20% bovine serum and 2 mM of glutamine were inoculated into 96 well plates. On the same day, DMSO solutions of the test compound B at final concentrations from 1 nM to 10 µM or only DMSO (DMSO group) were added thereto to culture the cells at 37 °C in 5% CO₂ for 4 days. Thereafter, the cell number was measured using a cell counting reagent (CellTiter-Glo (registered trademark) Luminescent Cell Viability Assay (Promega)). The cell proliferation inhibition rate was calculated as the measured value in the DMSO group set as 0% inhibition and the measured value in the well only with the medium on which the cells have not been seeded set as 100% inhibition.

As a result, the test compound B inhibited the proliferation of the CMK cells and with an IC₅₀ value of 110 nM.

### Example 12 Evaluation of ITK Kinase Inhibitory Activity

The ITK kinase inhibitory activity was evaluated using Mobility Shift Assay system.

The test compound B was dissolved in dimethyl sulfoxide (DMSO) to prepare a solution at a 100-fold concentration as compared to the test concentration. The solution was also 25-fold diluted with an assay buffer (20 mM HEPES, 0.01% Triton X-100, 1 mM DTT, pH 7.5) to obtain a test substance solution.

5 µL of the test compound B solution at a 4-fold concentration, prepared using the aforementioned assay buffer, and 10 µL of a ITK kinase solution at a 2-fold concentration were mixed within a well of a 384 well plate, and incubated at room temperature for 30 minutes. Continuously, 5 µL of a substrate peptide (Srctide, PEPTIDE INSTITUTE, INC. #AD-995)/ATP/MgCl₂ solution at a 4-fold concentration was added thereto to perform the reaction at room temperature for 1 hour. The substrate peptide was used at a final concentration of 1000 nM, ATP at a final concentration of 1000 µM, and MgCl₂ at a final concentration of 5 mM. Thereafter, 60 µL of a termination buffer (127 mM HEPES, 0.01 % Triton X-100, 26.7 mM EDTA, 1% DMSO, pH 7.5) was added thereto to stop the reaction. The ITK kinase activity was calculated by quantifying a product conversion ratio obtained from the substrate peptide peak height and the product (phosphorylated substrate peptide) peak height with LabChip3000 (PerkinElmer).

For the data analysis, by defining the average conversion ratio of a control well including all the reaction components as 0% inhibition and defining the average conversion ratio of a background well including all the reaction components except for the ITK kinase as 100% inhibition, the inhibition rate was calculated from the average conversion ratio of the wells for testing the respective test compound B.

As a result, inhibitory % of the test compound B at 1 nM and 10 nM against ITK kinase activity were 29.5% and 92.6%, respectively.

### Example 13 Evaluation of Proliferation Inhibition using Human Acute T Cell Lymphoma Cell Line Jurkat 6.1E Cells

Jurkat 6.1 E is a cell line originated from human acute T cell lymphoma and the ITK expression has been confirmed (Mol. Pharmacol. 2012; 82:938-947). The human acute T cell lymphoma cell line Jurkat 6.1E cells (European Collection of Cell Cultures, 88042803) cultured in RPMI1640 medium (Sigma) containing 10% bovine serum were seeded into 96 well plates. On the same day, DMSO solutions of the test compound B at a final concentrations from 1 nM to 10 µM or only DMSO (DMSO group) were added thereto to culture the cells at 37 °C in 5% CO₂ for 4 days. Thereafter, the cell number was measured using a cell counting reagent (CellTiter-Glo (registered trademark) Luminescent Cell Viability Assay (Promega)). The cell proliferation inhibition rate was calculated as the measured value in the DMSO group set as 0% inhibition and the measured value in the well only with the medium on which the cells have not been inoculated set 100% inhibition.

As a result, the test compound B inhibited the proliferation of the Jurkat 6.1E cells with an IC₅₀ value of 940 nM.

### Example 14 Evaluation of Proliferation Inhibition Using Human Lymphoblastic Leukemia Cell Line MOLT-4 Cells

MOLT-4 is a cell lines originated from human lymphoblastic leukemia and the ITK expression has been confirmed (Mol. Pharmacol. 2012; 82:938-947). The human lymphoblastic leukemia cell line MOLT-4 cells (European Collection of Cell Cultures, 85011413) cultured in RPMI1640 medium (Sigma) containing 10% bovine serum were inoculated into 96 well plates. On the same day, DMSO solutions of the test compound B at final concentrations from 1 nM to 10 µM or only DMSO (DMSO group) were added thereto to culture the cells at 37 °C in 5% CO₂ for 4 days. Thereafter, the cell number was measured using a cell counting reagent (CellTiter-Glo (registered trademark) Luminescent Cell Viability Assay (Promega)). The cell proliferation inhibition rate was calculated as the measured value in the DMSO group set as 0% inhibition and the measured value in the well only with the medium on which the cells have not been seeded set as 100% inhibition.

As a result, the test compound B inhibited the proliferation of the MOLT-4 cells with an IC₅₀ value of 410 nM.

From the above result, it is confirmed that the compound A or a pharmaceutically acceptable salt thereof inhibits activity of the BTK, JAK3 and ITK kinases and it is shown that use of the compound A or a pharmaceutically acceptable salt thereof as an active ingredient of the pharmaceutical composition for treating cancer in which one or more kinases of BTK, JAK3, and ITK is involved, can be expected. Specifically, it is confirmed that the compound A or a pharmaceutically acceptable salt thereof inhibits proliferation of the OCI-Ly10 cell which is a diffuse large B-cell lymphoma cell line, Rec-1 cell which is a mantle cell lymphoma cell line, KG-1 cell and MV4-11 cell which are acute myeloid leukemia cell lines, U-937 cell which is a histiocytic lymphoma cell line, SU-DHL-1 and KARPAS-299 cell which are anaplastic large cell lymphoma, CMK cell which is an acute megakaryocytic leukemia cell line, Jurkat 6.1E which is an acute T cell lymphoma cell line, and MOLT-4 cell which is a lymphoblastic leukemia cell line, and the compound A or a pharmaceutically acceptable salt thereof has an effect on cancer in which one or more kinases of BTK, JAK3, and ITK is involved. Further, even in the mouse model subcutaneously xenografted with OCI-Ly10 cells, a diffuse large B-cell lymphoma cell line, compound A monomethanesulfonate showed remarkable antitumor activity with regression in a dose-dependent manner.

### Industrial Applicability

It is expected that the compound A or a pharmaceutically acceptable salt thereof, which is an active ingredient of the pharmaceutical composition of the present invention, has an BTK inhibiting effect, a JAK3 inhibiting effect and an ITK inhibiting effect and can be used as an active ingredient of the pharmaceutical composition for treating cancer in which one or more kinases of BTK, JAK3 and ITK is involved, in one aspect, cancer in which BTK is overexpressed or activated, in another aspect, cancer in which a B cell receptor signal is activated, in still another aspect, cancer in which JAK3 is activation-mutated or activated, and in still another aspect, cancer in which ITK is activated.

## Claims

1. A pharmaceutical composition for treating cancer in which one or more kinases of BTK, JAK3, and ITK is involved, comprising 5-{[(3R)-1-acryloylpyrrolidin-3-yl]oxy} -6-ethyl-3-({4-[4-(4-methylpiperazin-1-yl)piperidin-1-yl]phenyl}amino)pyrazine-2-carboxamide or a pharmaceutically acceptable salt thereof as an active ingredient.

2. The pharmaceutical composition according to claim 1, wherein the cancer in which one or more kinases of BTK, JAK3, and ITK is involved is cancer in which BTK is involved.

3. The pharmaceutical composition according to claim 2, wherein the cancer in which BTK is involved is cancer in which BTK is overexpressed or activated.

4. The pharmaceutical composition according to claim 2, wherein the cancer in which BTK is involved is cancer in which a B cell receptor signal is activated.

5. The pharmaceutical composition according to claim 2, wherein the cancer in which BTK is involved is cancer selected from the group consisting of follicular lymphoma, chronic lymphocytic leukemia, small lymphocytic lymphoma, multiple myeloma, mantle cell lymphoma, diffuse large B-cell lymphoma, B-cell prolymphocytic leukemia, acute lymphocytic leukemia, histiocytic lymphoma, and acute myeloid leukemia.

6. The pharmaceutical composition according to claim 5, wherein the cancer in which BTK is involved is diffuse large B-cell lymphoma, mantle cell lymphoma, acute myeloid leukemia and/or histiocytic lymphoma.

7. The pharmaceutical composition according to claim 1, wherein the cancer in which one or more kinases of BTK, JAK3, and ITK is involved is cancer in which JAK3 is involved.

8. The pharmaceutical composition according to claim 7, wherein the cancer in which JAK3 is involved is cancer in which JAK3 is activation-mutated or activated.

9. The pharmaceutical composition according to claim 7, wherein the cancer in which JAK3 is involved is acute megakaryocytic leukemia and/or anaplastic large cell lymphoma.

10. The pharmaceutical composition according to claim 1, wherein the cancer in which one or more kinases of BTK, JAK3, and ITK is involved is cancer in which ITK is involved.

11. The pharmaceutical composition according to claim 10, wherein the cancer in which ITK is involved is cancer in which ITK is activated.

12. The pharmaceutical composition according to claim 10, wherein the cancer in which ITK is involved is acute T cell lymphoma and/or lymphoblastic leukemia.

13. The pharmaceutical composition according to claim 1, wherein the cancer in which one or more kinases of BTK, JAK3, and ITK is involved is cancer in which BTK and JAK3 are involved.

14. The pharmaceutical composition according to claim 1, wherein the cancer in which one or more kinases of BTK, JAK3, and ITK is involved is cancer in which BTK and ITK are involved.

15. The pharmaceutical composition according to claim 1, wherein the cancer in which one or more kinases of BTK, JAK3, and ITK is involved is cancer in which JAK3 and ITK are involved.

16. The pharmaceutical composition according to claim 1, wherein the cancer in which one or more kinases of BTK, JAK3, and ITK is involved is cancer in which BTK, JAK3 and ITK are involved.

17. The pharmaceutical composition according to any one of claims 1 to 16, wherein 5-{[(3R)-1-acryloylpyrrolidin-3-yl]oxy}-6-ethyl-3-({4-[4-(4-methylpiperazin-1-yl)piperidin-1-yl]phenyl}amino)pyrazine-2-carboxamide or a pharmaceutically acceptable salt thereof is 5-{[(3R)-1-acryloylpyrrolidin-3-yl]oxy}-6-ethyl-3-({4-[4-(4-methylpiperazin-1-yl)piperidin-1-yl]phenyl}amino)pyrazine-2-carboxamide monomethanesulfonate.

18. Use of the compound A or a pharmaceutically acceptable salt thereof for the manufacture of a pharmaceutical composition for treating cancer in which one or more kinases of BTK, JAK3, and ITK is involved.

19. Use of the compound A or a pharmaceutically acceptable salt thereof for treating cancer in which one or more kinases of BTK, JAK3, and ITK is involved.

20. The compound A or a pharmaceutically acceptable salt thereof for treating cancer in which one or more kinases of BTK, JAK3, and ITK is involved.

21. A method for treating cancer in which one or more kinases of BTK, JAK3, and ITK is involved, comprising administering an effective amount of the compound A or a pharmaceutically acceptable salt thereof to a subject.
